# EUROPEAN PATENT APPLICATION

(11) **EP 1 209 229 A1**
(43) Date of publication of application: **29.05.2002**
(21) Application number: 00125522.3
(22) Date of filing: 21.11.2000
(51) Int. Cl.: C12N 15/12, C07K 14/705, C12N 15/11, A61K 48/00, A61K 38/17, A61P 25/18, G01N 33/68, C12Q 1/68, A01K 67/027

(54) **Gene involved in schizophrenia**

(71) Applicant: Lesch, Klaus-Peter, Department of Psychiatry and Psychotherapy, University of Würzburg, 97080 Würzburg (DE); Meyer, Jobst, Department of Psychiatry and Psychotherapy, University of Würzburg, 97080 Würzburg (DE)
(72) Inventor: Lesch, Klaus-Peter, Department of Psychiatry and Psychotherapy, University of Würzburg, 97080 Würzburg (DE); Meyer, Jobst, Department of Psychiatry and Psychotherapy, University of Würzburg, 97080 Würzburg (DE)
(74) Representative: Becker Kurig Straus

(57) **Abstract**

The present invention pertains to a gene and the polypeptide encoded thereby. In particular, the present invention relates to mutations of said gene, which result in the development of schizophrenic symptoms. The present invention also relates to the use of said gene and its mutated forms for screening for biological active compounds having an impact on the development and/or existence of schizophrenia or related disorders in an individual and for the diasgnosis for an individual's tendency to develop schizophrenic symptoms. The present invention also relates to animal models for carrying out said assays.

## Description

The present invention pertains to a novel gene and the polypeptide encoded thereby. In particular, the present invention relates to mutations of said gene, which result in the development of schizophrenic symptoms. The present invention also relates to the use of said gene and its mutated forms for screening for biological active compounds having an impact on the development and/or existence of schizophrenia or related disorders in an individual and for the diagnosis of an individual's tendency to develop schizophrenic symptoms. The present invention also relates to animal models for carrying out said assays.

Schizophrenia and related disorders, which include schizo-affective disorders, bipolar disorders with schizophrenic features etc. are a complex and heterogeneous syndromes which encompass a variety of different symptoms. These symptoms are categorized in generic terms as being "positive", which comprises hallucinations, delusions, and conceptual disorganization and "negative", such as e.g. apathy, social withdrawal, affect, and poverty of speech. Schizophrenia afflicts a total of approximately 1 to 2% of all the population worldwide with its economical and social burden on society therefore being immense. In addition, since the onset of said disease generally occurs in 20-30 year old patients they are required to be under medical and psychiatric supervision for most of their lives.

Schizophrenia has been found to be associated with an abnormal activity of the neurotransmitter dopamine. Dopaminergic activity is reduced in the mesocortical system, which is deemed to result in negative symptoms and at the same time is enhanced in the mesolimbic system, which is considered to result in positive or psychotic symptoms. Also other neurotransmitters, including serotonin, glutamate and GABA are presently deemed to be implicated in said ailment.

For many years, schizophrenia has been treated with classical antipsychotic drugs, the so-called neuroleptics, that block central dopamine receptors. Neuroleptic drugs include compounds of the class of phenothiazines, piperidines, piperazines, diphenylpiperidines, etc., known representatives of which are chlorpromazine, thioridazine or pimozide.

However, these neuroleptics are effective for treating the positive symptoms of schizophrenia, but have little or no effect on the negative symptoms. In addition, most neuroleptics have been found to cause extrapyramidal symptoms, including rigidity, tremor, bradykinesia (slow movement), and bradyphrenia (slow thought), as well as tardive dyskinesias and dystonias.

In order to overcome these drawbacks a different class of antipsychotic drugs with a different receptor binding profile and effectiveness against the symptoms of schizophrenia have been developed. These compounds are summarized under the generic term "atypical antipsychotics"

In addition to dopamine receptors atypical antipsychotics also bind to central serotonin 2 (5-HT2) receptors. Unlike the conventional neuroleptics, they improve negative as well as positive symptoms and cause minimal extrapyramidal symptoms and only in rare cases tardive dyskinesias, akathisia, or acute dystonic reactions.

The first atypical antipsychotic drug approved for the treatment of schizophrenia was clozapine. Clozapine binds only weakly to D2 dopamine receptor, but exhibits a strong affinity for the 5-HT_{2A} receptor. Clozapine also antagonizes adrenergic, cholinergic, and histaminergic receptors. Clozapine has been found to be quite effective for the treatment of schizophrenia, especially for subjects who do not respond to traditional neuroleptic therapy and this compound is generally well tolerated.

However, clozapine shows severe side effects, including sedation, orthostatic hypotension, hypersalivation, lowered seizure threshold and, in particular, agranulocytosis. Agranulocytosis is a serious condition associated with a substantial decrease in white blood cell count. For these reasons, patients taking clozapine have to be monitored for their white blood cell number on a regular basis. Another drawback of this compound is the relatively short half life in vivo, which necessitates administering multiple doses daily to maintain therapeutic effectiveness.

Consequently, there is a need in the art for improved means and methods for treating schizophrenia.

An object of the present invention therefore resides in providing such means and methods and to enable a better understanding of the genetic basis and pathogenesis for schizophrenia.

The above problem has been solved according to a first aspect by providing a nucleic acid as identified by SEQ. ID No. 1, or the coding region thereof, respectively, and mutational variants thereof, wherein one or more nucleotides are substituted or deleted such that the polypeptide encoded thereby retains its biological function.

According to a second aspect the invention provides for a nucleic acid, identified by SEQ. ID. No. 1, which has been modified by adding, substituting or deleting one or more nucleic acids such that the biological function of the polypeptide encoded thereby is essentially disturbed. Examples for such nucleic acids are truncated nucleic acids (introduction of an early stop codon) to be used as dominant-negative inhibitors. According to a preferred embodiment the nucleic acid is identified by SEQ. ID. No. 2.

According to a third aspect the present invention provides for a polypeptide encoded by the nucleic acids mentioned above and in particular a polypeptide as identified by SEQ ID. No. 3 or SEQ. ID. No. 4.

Further the present invention provides a genomic sequence as identified by SEQ. ID. No. 5 coding for the WKL-1 polypeptide. The Intron-Exon pattern is as follows: Exon 1: nt 8526-8663; Exon 2: nt 9056- 9291; Exon 3: nt 10844-10933; Exon 4: nt 13620-13673; Exon 5: nt 13998-14099; Exon 6: nt 16515-16616; Exon 7: nt 17105-17176; Exon 8: nt 19685-19801; Exon 9: nt 23434-23490; Exon 10: nt 25462-25584; Exon 11: nt 29819-29983; Exon 12: nt 32360-34626.

In addition, the present invention also provides a pharmaceutical composition containing a nucleic acid as described herein or a polypeptide encoded thereby.

The present invention also pertains to models for screening for biologically active substances for treating schizophrenia or related diseases (e.g. schizo-affective disorders or bipolar disorders), such as animal models or cell culture models.

Moreover, the invention also relates to the use of said nucleic acids and polypeptides and to models for developing drugs for treating schizophrenic symptoms and related disorders.

In the figures,
Fig. 1 shows a survey of a pedigree of a large German family co-segregating a C1121A mutation in the gene with periodic catatonia, a familial subtype of catatonic schizophrenia. Affected members are symbolized by black boxes (males) or circles (females). Alleles of two adjacent polymorphic markers are provided (D22S1161 and D22S526). Assumed haplotypes of deceased persons are given in parenthesis;
Fig. 2 shows an autoradiogramm of a northern blot showing gene expression of WKL-1 in various brain regions and peripheral tissues;
Fig. 3 shows a Kyte / Doolittle hydrophilicity plot and a Robson Gamier secondary structure assessment of the original polypeptide;
Fig. 4 shows a Kyte / Doolittle hydrophilicity plot and a Robson Gamier secondary structure assessment of the mutated polypeptide; and

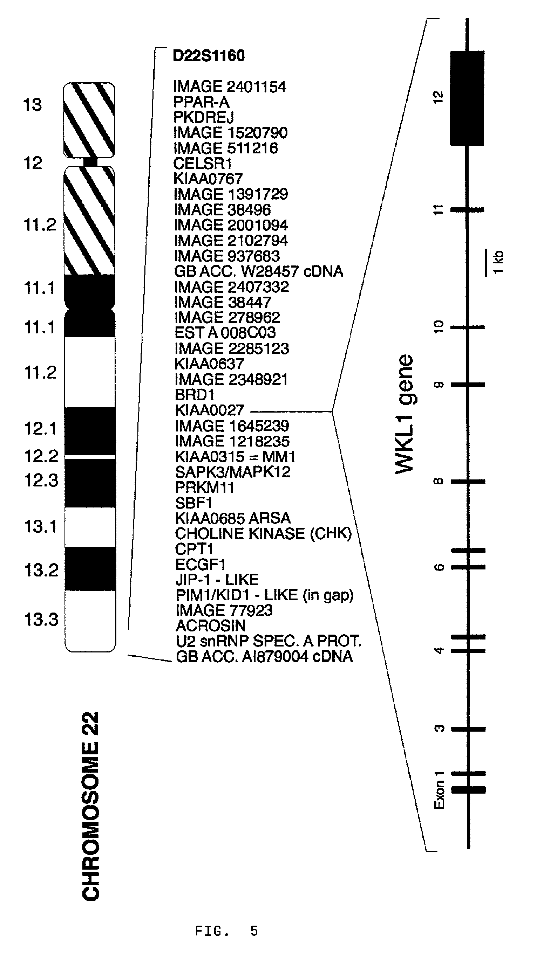

Fig. 5 shows a genomic map of the WKL-1 gene.

In the various studies leading to the present invention the inventors have investigated a pedigree of individuals suffering in part from catatonic schizophrenia, in particular from periodic catatonia. Periodic catatonia, a familial subtype of catatonic schizophrenia, is a disorder characterized by psychosis and psychomotor disturbances. Patients with periodic catatonia express a variable phenotype combining akinetic negativism, hyperkinesia with stereotypies and parakinetic movements as well as increased anxiety, impulsivity, and aggressiveness. Acute psychotic episodes may be accompanied by hallucinations and delusions, while successive episodes lead to an increasingly severe catatonic residual state.

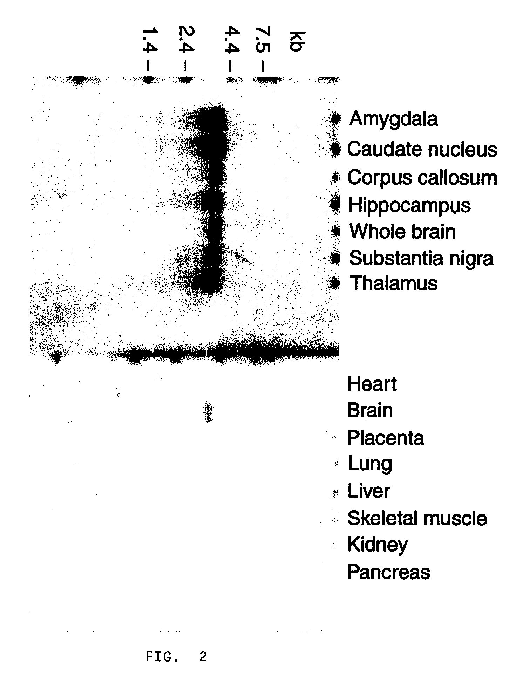

The present inventors have now found that a mutation in a specific, not yet characterized gene, termed WKL-1, co-segregates with the disease condition in the individuals investigated. The gene identified yields a transcript of approximately 3.6 kb in length. According to Northern blot analysis (see Fig. 2) transcripts of this gene are detected exclusively in human brain with highest signal intensities found in the amygdala, nucleus caudatus, thalamus, and hippocampus. The open reading frame of the cDNA obtained yields a polypeptide having 377 amino acids.

A comparison of the amino-acid sequence with known proteins identifies said polypeptide as a potassium channel with 6 putative transmembrane regions and segments in between forming a hairpin structure. Highest similarity was obtained for the shaker related human voltage-gated potassium channel KCNA1 (Browne, D. et al., Nat. Genet. **8** (1994), 136-140) with the similarity being most pronounced in the transmembrane domaines.

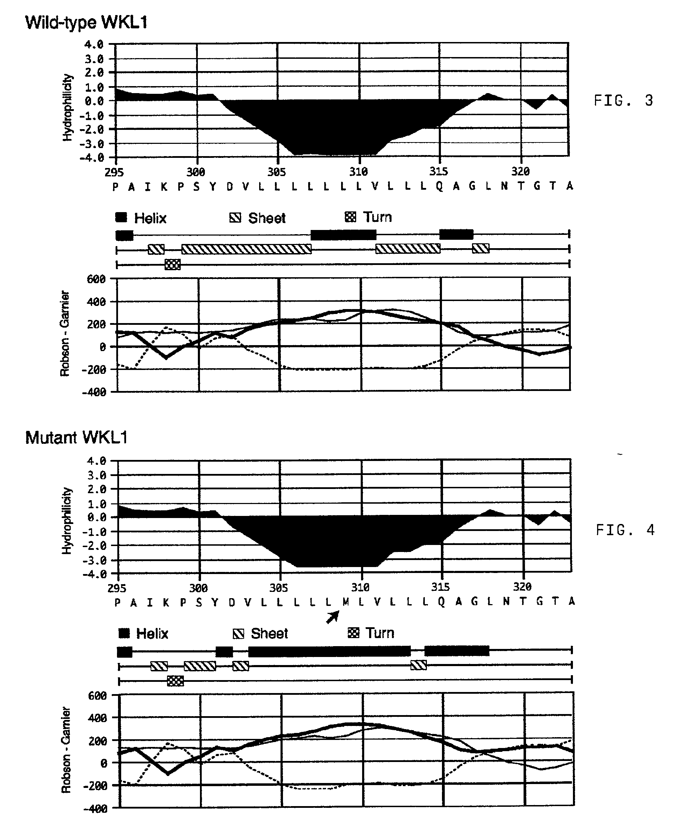

An analysis of the WKL-1 gene isolated from all affected family members and a total of 654 chromosomes derived from controls exhibited a mutation in the DNA sequence leading to an amino acid exchange at position 309 from leucine to methionine. According to a structural analysis this exchange alters the three dimensional conformation of the polypeptide as shown in Fig. 4 and adversely influences the normal functioning of the potassium channel eventually resulting in the development of clinical symptoms associated with schizophrenic conditions.

This notion is supported by findings of mutations in other potassium channel encoding genes, such as in KCNA1, causing episodic (periodic) ataxia (Browne, D. et al., supra) and in KCNQ2, which leads to neonatal human epilepsy (Biervert, C. et al., Science **279** (1998), 403-406).

In addition, high expression of mutant WKL-1 in the nigro-striatal motor and mesolimbic systems leading to restricted channel function in these brain regions would also be in agreement with prominent psychopathologic features of periodic catatonia, such as psychomotor dysfunction, impaired cognitive functioning, and disinhibited or attenuated affective states.

According to a preferred embodiment the invention provides a pharmaceutical composition containing a nucleic acid encoding the native, i.e. the non-mutated WKL-1 polypeptide. This sort of pharmaceutical composition aims at eventually increasing the level of the original polypeptide in the cells.

To this end, a nucleic acid coding for the native (non-mutated) gene product (representing cDNA or genomic sequence), that is a polypeptide as represented by SEQ. ID. No. 3, is integrated in a suitable vector and is introduced into somatic cells of an affected individual.

This may be achieved either by means of DNA-mediated gene transfer, which involves direct administration of the corresponding DNA to the patient in various formulations. These methods use the genes as medicines in a manner much like conventional organic or protein compounds. Alternatively, the nucleic acid of interest may be introduced into somatic cells by means of viral gene transfer, which involves construction of synthetic virus particles (vectors) that lack pathogenic functions. The virus particles are generally incapable of replication and contain the therapeutic gene within the viral genome which is delivered to cells by the process of infection. As examples for such viral vectors a retroviral vector derived from Moloney Murine Leukemia Virus capable of permanently integrating the genes they carry into the chromosomes of the target cell, vectors based on adenovirus nucleic sequences or vectors based on the Herpes Simplex virus may be mentioned. Herpes virus vectors are capable of infecting cells and persisting indefinitely in a latent state. Traditionally, the Herpes Simplex virus vector involves genetic engineering of the viral genome to render it useful for serial propagation and for sustained expression of foreign genes in a suitable host. In addition, other components may also be added such as the Epstein Barr virus nuclear antigen gene and latent origin of replication to maintain the vector in an episomal state.

Due to the host range and efficient genomic integration resulting in chronic transgene expression retroviruses including the subclass of lentiviruses are preferred vectors for stable gene transfer into terminally differentiated cells such as neurons. More preferred vectors are derived from the human immune deficiency virus (HIV) which may be used to target post-mitotic neurons. These vectors infect the cell, transport the transgene actively through the nuclear pore, and since they are devoid of genes encoding the HIV envelope, they alternatively express the vesicular stomatitis virus protein envelope.

Once the WKL-1 gene has been introduced into somatic cells of an individual a tissue-specific transcription must be ensured, which may be accomplished by means of linking the nucleic acid encoding the WKL-1 polypeptide with a promoter, which is exclusively active in brain tissues or specific neuronal systems. The promoter and neighboring regulatory elements are located adjacent, preferably upstream of the gene to be transcribed. In contrast to constitutive promoters (e.g. viral long terminal repeats, LTR, or immediate early promoter of the cytomegaly virus, CMV-IE) which effectively drive expression in a large variety of tissues, tissue-selective regulatory elements confer cell-restricted actitvity such that the transferred gene is transcribed only in distinct neurons or glial cells. Examples for such promoters are promoters of the neuron-specific enolase (NSE) and glial acidic-fibrillary protein (GFAP) genes, which are currently used to express transgenes in neurons and astrocytes, respectively. Neurotransmitter systems, such as the serotonergic system, may be targeted e.g. with promoters of the L-tryptophane hydroxylase (L-TPH) or serotonin transporter (5-HTT) gene. Since the promoter/transgene construct is incorporated into the genome and thus present in all cell types, known transgenic techniques may be used for confirmation of cell or neuronal system-selective expression of the transferred gene.

While the above described instrument is designed to increase the level of the original WKL-1 polypeptide in brain tissues as such, this may also be achieved by administering the polypeptide as such in a suitable galenic form, so as to reach the corresponding target cells.

Alternatively, the level of the mutated polypeptides in brain tissues may be reduced by making use of e.g. antisense oligonucleotides or cRNA. Consequently, in an alternative embodiment the pharmaceutical composition may contain an antisense oligonucleotide/cRNA capable of reducing or inhibiting translation etc. of the mutated WKL-1 polypeptide.

Without being bound to any theory it is believed that the potassium channel discussed herein is comprised of a tetramer of the WKL-1 polypeptides as previously described in shaker-related voltage-dependent potassium channels. This notion also explains the incidence of the disease condition in some individuals which are heterozygous for the mutated gene, while other individuals, also heterozygous for said mutated gene, do not show symptoms of schizophrenia. This may be explained on the basis that the original (not mutated) allele in a heterozygous individual dominates the mutated allele, e.g. by means of an increased expression over the mutated form, eventually leading to an assembly of the potassium channel mainly being comprised by the original WKL-1 polypeptides and forming a normally functional channel. If, however, in individuals heterozygous for the original and mutated WKL-1, the transcription, expression etc. of the mutated form exceeds that of the original form, potassium channels are assembled that exhibit an adversely affected biological function.

In consequence, when reducing the level of the mutated transcript in the cells an assembly of the potassium channels will primarily be effected with the the native, i.e. non-mutated form of the WKL-1 polypeptide.

Therefore the present invention also provides a pharmaceutical composition containing oligonucleotides or cRNA (e.g. RNA interference) as therapeutic agents that are antisense to a region of the WKL-1 gene that harbors the mutation, responsible for the biological malfunction. The term oligonucleotide as used herein includes linear oligomers of natural or modified monomers or linkages, including desoxyribonucleosides, ribonucleosides, α-anomeric forms thereof, polyamide nucleic acids, and the like, capable of specifically binding to the target nucleic acid by way of a regular pattern of monomer-to-monomer interactions, such as Watson-Crick type of base pairing, Hoogsteen or reverse Hoogsteen types of base pairing, or the like.

Usually the monomers are linked by phosphodiester bonds or analogs thereof to form oligonucleotides ranging in size from a few monomeric units, e.g. 3-4, to several 10 of monomeric units, depending on the size or region of mutation. The antisense oligonucleotides may also contain pendent groups or moieties, to enhance specificity, nuclease resistance, delivery, or other property related to efficacy, such as e.g. cholesterol moieties, duplex intercalators such as acridine, poly-L-lysine, "end capping" with one or more nuclease-resistant linkage groups such as phosphorothioate, and the like.

By administering a therapeutically effective amount of an antisense compound to a tissue having a target polynucleotide the inappropriate synthesis of the mutated WKL-1 protein will be reduced or substantially inhibited.

The pharmaceutical composition may also comprise an effective amount of a WKL-1 polypeptide or a functional part thereof.

In all of the three embodiments described above (nucleic acid coding for the native polypeptide, the polypeptide as such, antisense oligonucleotides) the pharmaceutical composition will further contain carriers and excipients that are suitable for delivering the respective active molecule of different nature to the target tissue. The kind of the carrier/excipient and the amount thereof will depend on the nature of the substance and the mode of drug delivery and/or administration contemplated.

E.g., for formulations containing weakly soluble antisense oligonucleotides, microemulsions may be employed, for example by using a non-ionic surfactant such as Tween 80 in an amount of about 0.04-0.05% (w/v), to increase solubility. Other components may include antioxidants, such as ascorbic acid, hydrophilic polymers, such as, monosaccharides, disaccharides, and other carbohydrates including cellulose or its derivatives, dextrins, chelating agents, such as EDTA, and like components well known to those in the pharmaceutical sciences.

These various components utilized provide a variety of functions, including regulation of drug concentration, regulation of solubility, chemical stabilization, regulation of viscosity, absorption enhancement, regulation of pH, and the like. For example, in water soluble formulations the pharmaceutical composition preferably includes a buffer such as a phosphate buffer, or other organic acid salts, preferably at a pH of between about 7 and 8.

It will be appreciated that the skilled person will, based on his own knowledge select the appropriate components to target the active compound to the brain tissue taking into account the route of administration which may be by way of injection, topical application, intranasal administration, administration by implanted or transdermal sustained release systems, and the like.

According to another preferred embodiment the present invention also contemplates the use of the nucleic acids encoding the WKL-1 polypeptides and the various mutated variants thereof for screening for biologically active compounds that positively affect disease conditions associated with schizophrenia or related disorders, in particular catatonic schizophrenia.

In this respect model systems for such screening assays may be developed, such as e.g. by introducing a nucleic acid encoding the original or a mutated form of the WKL-1 polypeptide into e.g. Xenopus oocytes. This model is quite suited for carrying out experiments for assessing protein function, such as e.g. performing voltage clamp analyses in order to elucidate the function of the protein (potassium channel) and to be able to understand the way it works. When understanding the functionality of the channel on the molecular level appropriate drugs interacting with the channel may be designed on the computer.

Alternatively, a nucleic acid encoding a mutated form of the WKL-1 polypeptide may be introduced into mammalian cells, preferably neuronal progenitor cells, making use of the phenomenon of homologous recombination. In this respect cells of a primary culture of mammalian cells may be immortalized by means well known in the art, such as e.g. by using the large T-antigen of SV40 or the technique involving the reverse transcriptase of the telomerase, with said immortalized cells maintaining their inherent properties. With the cell lines thus obtained the endogenous WKL-1 gene may be knocked out by introducing a mutated form thereof, that lacks normal functioning, preferably by introducing a nucleic acid as identified by SEQ. ID. No. 2, making use of the phenomenon of homologous recombination. The construct with which the mutated form of the WKL-1 polypeptide is introduced into the cell may also contain selection markers, so as to facilitate selection for cells, into the genome of which the constructs has been integrated. Such selection markers are widely spread in the art and may e.g. be an antibiotic resistance.

Cells containing the construct may then be assayed for the presence of the mutated form and the concurrent absence of the original form. Likewise, in case a cell is desired heterozygous for the original and the mutated form, the cells may be assayed for the presence of both transcripts.

When such a cell line has been established said cell line is a perfect tool for screening for compounds restoring the normal functioning of the potassium channel as may be evidenced by immortalized brain cells, kept in culture.

Alternatively, transgenic non-human animals may be prepared, which may be knock-in animals, that is animals, into which the desired gene function has been introduced (the mutated form of the WKL-1 polypeptide leading to an impaired function of the potassium channel) or a knock-out animal, an animal that lacks the gene function due to e.g. disruption of the gene. Methods and techniques for preparing transgenic animals are well known in the art.

These non-human animals may well be used for studying the effect of the absence of the potassium channel as such (knock-out animal), which may also involve collecting the animals at an embryonic state, in case the animals die before birth. In addition, in knock-in animals the molecular effect of the mutated form of the WKL-1 polypeptide may be studied and said animals may be used for screening for biological active compounds positively influencing the normal functioning of the potassium channel. Such an animal model also enables the skilled person to evaluate potential side effects of compounds found to influence the function of the potassium channel discussed herein. Such biological active compound will be a compound controlling the ion flux through the potassium channel.

The invention also relates to the transgenic cells and cell lines and transgenic non-human animals as described herein. The animals are preferably of mammalian origin and are preferably mouse, rat or non-human primates, such as rhesus monkey.

In addition the present invention also provides for a means for diagnosing an individual's tendency to develop schizophrenic symptoms. In this respect, a sample is taken from an individual to be examined and the gene coding for WKL-1 is investigated whether it corresponds to the native, i.e. non-mutated form or a mutated form. Once a sample is taken this may easily be achieved by examining the individual's DNA for known mutations or by amplifying specifically the exons of the gene and investigating the exons for the presence of a mutation. Nowadays there is a huge variety of different tools available to check for the existence of a mutation in a DNA sequence.

Alternatively, it has also been found that the WKL-polypeptide is expressed in lymphocytes as well, so that an assay based on a blood sample may be performed. In this respect blood is taken from an individual and the cellular material, including the lymphocytes is isolated. The mRNA of the cellular material is isolated and an assay is performed, for detecting the presence/absence of a given mutation. This may be implemented by e.g. RT-PCR or other means, wherein the primers are selected such so as to be located on different exons, so as to produce a fragment only in case a mRNA is present. The fragment may then be sequenced in a manner known to the skilled person. Alternatively, one of the primers used for performing the assay binds to the location on which the mutation is presumed, only in the case the mutation is present/absent. This may be achieved by selecting the nucleotide sequence of the primer according to the nucleotide sequence of the native (non-mutated, encoding a fully functional polypeptide) gene or the nucleotide sequence according to the mutated gene. It will be appreciated that the skilled person will based on his own technical skill select the appropriate oligonucleotide as primer.

The following examples illustrate the invention without limiting it thereto.

### Examples

### Example 1

A genome-wide linkage scan of 12 German pedigrees with the periodic subtype catatonic schizophrenia comprising 137 individuals and 57 affected was carried out. Microsatellite markers were selected from the human genetic map. Three hundred fifty-six microsatellite markers were chosen for regular spacing throughout the genome, including markers at the most telomeric positions. The markers had a mean sex-averaged spacing of approximately 11 cM and an average heterozygosity of 0.78 (range 0.51 - 0.92). Marker amplification was performed in microtiter plates on thermocyclers, and PCR pools were separated on ABI 377 automatic sequencers. Genotyping was done by operators blind to phenotype. Allele calling was checked by operational procedures and was subject to an automated Mendelian check using the UNKNOWN program from the LINKAGE program package. Allele sizes were standardized those of known CEPH control individuals. For a total of 47.655 genotypes, the effective typing ratio was 96.5%.

Nonparametric, model-free multipoint linkage analyses were performed with the GENEHUNTER-PLUS (GH-PLUS) package. GH-PLUS allows pedigree analysis of complex inherited traits, and it estimates the statistical significance of shared alleles identical-by-descent between all affected members of the pedigree. The GH-PLUS modification permits exact calculation of likelihood ratios on the basis of an exponential allele-sharing model. P values can be approximated by applying normal approximation. The nonparametric LOD scores (LOD*), based on the allele sharing, can be used within the same inferential framework as is used for traditional LOD scores. Maximum two-point homogeneity LOD scores were calculated using the MLINK program of the FASTLINK software package, version 5.1, and tests of heterogeneity were performed using the HOMOG program, version 3.35. For computation of parametric multipoint likelihoods, four-point LOD-score analyses were performed, using the program VITESSE, with three adjacent pairs of screening-set markers and the disease locus. Intermarker distances were derived from the Genethon map. Analyses were performed with affected individuals only (setting those not affected to unknown), under an autosomal dominant model (penetrance vector 0.0, 1.0, 1.0) and a disease-allele frequency of .001; phenocopy rate was set at 0.0.

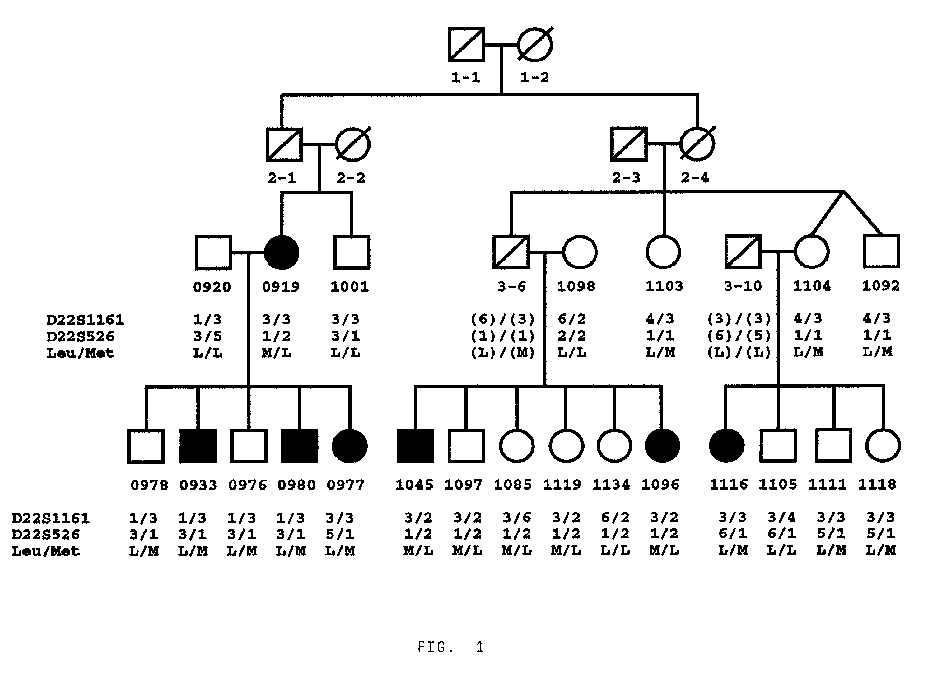

In this group of individuals, significant evidence for linkage was obtained on chromosome 15 by using non-parametric multipoint analysis methods (GENEHUNTER-PLUS LOD* score 3.57, p=0.000026). For a second locus, mainly supported by a single large family (family 21, Fig. 1), suggestive evidence for linkage was found for marker D22S1169 on chromosome 22q13.33 (LOD* score 1.85, p=0.0018).

Genotyping of additional chromosome 22-specific polymorphic markers (D22S1160, D22S1149, D22S1170, D22S1161, D22S526) restricted the region of interest to approximately 4 cM extending from D22S1160 to the telomer. Marker amplification was performed in thermocyclers, and PCR products were separated on ABI 377 automatic sequencers. Genotyping was done by operators blind to phenotype.

A partial cDNA (provisionally termed KIAA0027) deposited in GenBank with a short, potentially unstable CTG stretch located within this region was among several strong positional candidates that were selected for further investigation.

In order to screen for gene variants leading to the schizophrenic phenotype the 12 exons of KWL-1 were amplified from genomic DNA of two affected individuals of family 21 and three unrelated controls by PCR. Analysis of PCR product size of exon 11 containing the CTG stretch did not show evidence for length variability of the nucleotide sequence. However, sequencing demonstrated heterozygosity for a 1121C→A transversion in both patients, resulting in the amino acid substitution Leu309Met.

C1121A was genotyped by the creation of a *Sfa*NI restriction site in the mutant allele in the rest of the pedigree and in 654 chromosomes of controls. Seven affected individuals and obligate carriers were heterozygous for the mutation, while the 6 unaffected family members carry two wild-type alleles (Fig. 1).

The mutation was found to co-segregate with the syndrome in the pedigree and in accordance with the haplotypes previously determined by the linkage scan, whereas C1121A was not detected in the controls. Screening of three additional families with confirmed inheritance of the chromosome 22-specific haplotypes for mutations in the entire WKL-1 gene (coding and non-coding regions, intron/exon boundaries) failed to detect C1121A or alternative mutations. The presence of the mutation in asymptomatic individuals was interpreted as examples of the previously reported incomplete penetrance or atypical and relatively mild course of illness under, as yet, unspecified circumstances.

The 5'-UTR of WKL-1 was extended by 5'-RACE to an overall cDNA length of 3515 base pairs. In the expanded 5'-UTR a stop codon (TGA) was found in the previously reported open reading frame. The ATG codon at nucleotide position 197 is to be considered as the correct translation initiation site, resulting in a protein of 377 amino-acid residues (SEQ. ID. No. 3).

A genomic annotation of the cDNA revealed 12 exon spanning about 28 Mb. The exon boundaries are in SEQ ID. No. 5: Exon 1: nt 8526- 8663; Exon 2: nt 9056- 9291; Exon 3: nt 10844-10933; Exon 4: nt 13620-13673; Exon 5: nt 13998-14099; Exon 6: nt 16515-16616; Exon 7: nt 17105-17176; Exon 8: nt 19685-19801; Exon 9: nt 23434-23490; Exon 10: nt 25462-25584; Exon 11: nt 29819-29983; Exon 12: nt 32360-34626.

Northern blots of WKL-1 mRNA revealed a single band with a size of approximately 3.6 kilobases, and transcripts were detected exclusively in human brain with highest signal intensities found in the amygdala, nucleus caudatus, thalamus, and hippocampus. The size of the WKL-1 transcript is in agreement the results obtained by the 5'-RACE technique.

More detailed assessment of WKL-1 expression using both Northern and in situ hybridisation of human postmortem brain demonstrated transcripts also in substantia nigra, putamen, and cerebellum. No detectable expression was found in peripheral tissues including heart and skeletal muscle.

Analysis of the revised version of the deducted WKL-1 protein revealed 6 putative transmembrane domains (S1 - S6) with the segment between S5 and S6 forming a hairpin structure extending into the membrane. As expected from these features, highest similarity scores were obtained for the shaker-related human voltage-gated potassium channel KCNA1 (average approx. 24%) followed by other members (Kv1.1 - Kv1.10) of this extended gene family. The similarity is most pronounced in the transmembrane domaines S2 - S5. These characteristics suggest that the WKL-1 protein may act as a neuronal potassium channel, and thus represents the first member of a novel subfamily related to the shaker superfamily.

Potassium channels are critical for repolarizing and thus termination of action potentials. Dysfunction of voltage-gated postassium channels results in impaired repolarization and increased excitability of neurons. Based on the structure analysis the Leu309Met mutation is located in the transmembrane domain S6 resulting in alterations of the secondary structure of WKL-1 including decreased hydrophilicity and helix/sheet conformation (Fig. 4). Due to the conformational changes observed the Leu309Met mutation compromises channel function which, in turn, leads to neuronal hyperexcitability. Since mutations in other potassium channel encoding genes such as KCNA1 cause episodic (paroxysmal) ataxia (supra), and KCNQ2 benign familial neonatal epilepsy (supra), episodic psychomotor derangements such as hyperkinesia and parakinetic movements observed in periodic catatonia supports the notion that WKL-1 encodes a potassium channel. High expression of mutant WKL-1 in the nigro-striatal motor and mesolimbic systems leading to restricted channel function in these brain regions is also in agreement with prominent psychopathologic features of periodic catatonia, such as psychomotor dysfunction, impaired cognitive functioning, and disinhibited or attenuated affective states.

SEQUENCE LISTING

<110> Lesch, Klaus, Peter
      Meyer, Jobst

<120> Novel gene involved in schizophrenia

<130> 80282

<140>
<141>

<160> 5

<170> PatentIn Ver. 2.1

<210> 1
<211> 3515
<212> DNA
<213> human

<400> 1
attgatattc aggaagcagc tctccttccc ctgccttcag ctcaagtttg ctgagctttt 60
gtttcatttg tgaatacttc ttgctggaag tccctcaccc agagaccagt gctcccaacg 120
gcagagcagc gggggagata aagaactggt gacacgtggc tgtacattca gcacagctgt 180
ggtgtcccca agtgccatga cccaggagcc attcagagag gagctggcct atgaccggat 240
gcccacgctg gagcggggcc ggcaagaccc cgccagctat gccccagacg cgaagccgag 300
cgacctgcag ctgtcgaaga gactgccccc ctgcttcagc cacaagacgt gggtcttctc 360
tgtgctgatg gggagctgcc tcctggtgac ctcggggttt tcgctgtacc tggggaacgt 420
gttcccggct gagatggatt acttgcgctg tgctgcaggc tcttgcatcc cctcggcaat 480
tgtgagcttc accgtctcca ggaggaacgc caatgtgatt cccaactttc agatattgtt 540
tgtttccacg tttgctgtga ccactacgtg tttaatttgg tttggatgca aactagtcct 600
gaacccatca gcaataaaca tcaacttcaa cctcatcctg ctgctcctgc tggagctgct 660
catggcggcc acggtgatca tcgctgcacg gtccagcgag gaggactgca agaaaaagaa 720
gggctccatg tctgacagcg ccaacattct ggacgaagtg ccatttcctg ctcgggtcct 780
gaaatcttac tcagtcgtcg aggtaatcgc aggcatctct gccgtcctcg gggggatcat 840
tgccctgaac gtggatgact cagtttcagg cccacacctc tcagtgacgt tcttttggat 900
cctagtggcc tgctttccaa gtgccattgc cagtcatgtg gcagcagagt gtcccagcaa 960
gtgtctggtg gaggtcctga ttgccataag cagcctcacg tctccgctgc tgttcacagc 1020
ctctggatat ctgtcattca gcatcatgag aatcgtggag atgtttaagg attacccgcc 1080
agccataaaa ccatcctacg atgtgctgct gctgctgctg ctgctagtgc tcctgctgca 1140
ggccggcctc aacacgggca ccgccatcca gtgcgtgcgc ttcaaggtca gtgcaaggct 1200
gcagggtgca tcctgggaca cccagaacgg cccgcaggag cgcctggctg gggaggtggc 1260
caggagcccc ctgaaggagt tcgacaagga gaaagcctgg agagccgtcg tggtgcaaat 1320
ggcccagtga cccccagacg cggaaaccgg gtggcagcgc ccagcctggc cccaagcatg 1380
gaaacgcaca acccctaatc gccctgagct actgcttcta acacctcttt tcccttgtgt 1440
gagggcaaac caggctgcag gtggggtttt cacttcctag ggtagtttaa ttttaaaata 1500
ggccaatgtt ggctagtctg tgcctcagtg agatcagtca gctccgagtg gctcccgtgt 1560

cgtaacagca ggagcatggc cgcaacttcc caggccgagg aagggccccc ggctcggcct 1620
cttgagagcc ccacccctga actggcccca gctcctcttc ctgcctctct catggcttgg 1680
gctggagtgg gctctctgga cctgaccaga ctgtgggtcc ctgcgtctcc tgcccactct 1740
gaccgggctt cctccctcca cgcttagggt ctgtcccggg tactcagtca gcccagtggg 1800
atcttaccca cttccctgca aggtgcacct gccccaggct caggctgccc agcggctctt 1860
cctggacagt gagagcaggg cttggcgcct ctgtcctggc ccgggagccg caggggcccc 1920
tcctccagag cctgggcgca agcgacacag gctgccgctg ctctcccagg tgaaatccac 1980
accagtccac gccgggtcgc ctgccctgtc tccctactta gacccagtca ttctagaggg 2040
atccaccgcc acactggccg gcccacgtcc tgggtgctgt catgcccagc ttggagtgcc 2100
acgtggccgc tgcccacgtc ccgggcactg tcatgcccag cttggagtgc cacatggccg 2160
ctgcccacgt cccgggcact gtcatgccca gcttggagtg ccacgtggcc gctgctgtga 2220
caggcagtgt tcttgggggt ggggctgcat ccaaggcttt gtaaaccggc tggaccacgt 2280
ctccctggcc ccagtgaccg ggggaagctg agcccctccc tcctgtgttt gctcccatta 2340
ctcaaaatgc aggacagatc aggtcagagc ccaggaattc tcacaggttc acccagcgcc 2400
ctctacctcc tagcaagtac tttgtcttga tcctcactga gaaggcccca gggcagcggt 2460
cttctccatc tccgctgttt tggggtctta gggtacagcc caggcggtca ctgcccacct 2520
gccaggctgc agggacagtt gggtgtgaga ataacactgg ctttgggtag tgccatggcc 2580
aggagtgggt ttccctgcgt ctcctcgtcc cgagggcgcc tgggtcctcc cagctgacgg 2640
cagtaaatcc acagtgagtt ggggcgactg tgaaactgga atgctgttac tttgataatt 2700
actttccagc aggtgttttc cttcacaatg gttttgtttc tttccttctg atctgagaag 2760
acatgaacgt tttctcttca ccgccgtggg gtgtattgac tggtccccca tgggctgctg 2820
gaaaggcccg gagatgcatc tgtggcctgg ggccatcaag atcaaagaac caggaggcct 2880
gggagatgca gctggatggg gcggcctgca gaccctgcca gggggtttga ggaccctccc 2940
aggtttccca ctgcggaaca ggagtgactc tggctgccaa gataccttca tggtgttcat 3000
gacaagtgga atcattattt tcaaccattg aagggggatg caggcaagac accttcccag 3060
ctgctcctag aggggacaag ccaggccctc tctgcagtcc tcggcagctc cggaaggaca 3120
cagtcagggg ccgggcaaac actttggcca cagccccaaa caagcgccac cgtgggagag 3180
gagaggctgc tgtcactggt accggatgca gaccccaccc tgtctgcagg ccacccccac 3240
ctccctgcag ctttgaggct ggcggggtct gctcctggga atggggtggg agccacaggg 3300
acgacccggg gcgggctgat gtcttcttgg gggcagacca gagagctcaa gtttcagagt 3360
cagaattagg cacttggaac gtttttgctg gcttgcactt tcttattttc ttattttaga 3420
gcgcttaaaa aatccggaaa aatggggttt aaaagaactg tctctttcag tctacatttt 3480
tgtttaatac gcttgagcaa taaacgctga cttgc 3515

<210> 2
<211> 3515
<212> DNA
<213> human

<400> 2
attgatattc aggaagcagc tctccttccc ctgccttcag ctcaagtttg ctgagctttt 60
gtttcatttg tgaatacttc ttgctggaag tccctcaccc agagaccagt gctcccaacg 120
gcagagcagc gggggagata aagaactggt gacacgtggc tgtacattca gcacagctgt 180
ggtgtcccca agtgccatga cccaggagcc attcagagag gagctggcct atgaccggat 240
gcccacgctg gagcggggcc ggcaagaccc cgccagctat gccccagacg cgaagccgag 300
cgacctgcag ctgtcgaaga gactgccccc ctgcttcagc cacaagacgt gggtcttctc 360
tgtgctgatg gggagctgcc tcctggtgac ctcggggttt tcgctgtacc tggggaacgt 420

gttcccggct gagatggatt acttgcgctg tgctgcaggc tcttgcatcc cctcggcaat 480
tgtgagcttc accgtctcca ggaggaacgc caatgtgatt cccaactttc agatattgtt 540
tgtttccacg tttgctgtga ccactacgtg tttaatttgg tttggatgca aactagtcct 600
gaacccatca gcaataaaca tcaacttcaa cctcatcctg ctgctcctgc tggagctgct 660
catggcggcc acggtgatca tcgctgcacg gtccagcgag gaggactgca agaaaaagaa 720
gggctccatg tctgacagcg ccaacattct ggacgaagtg ccatttcctg ctcgggtcct 780
gaaatcttac tcagtcgtcg aggtaatcgc aggcatctct gccgtcctcg gggggatcat 840
tgccctgaac gtggatgact cagtttcagg cccacacctc tcagtgacgt tcttttggat 900
cctagtggcc tgctttccaa gtgccattgc cagtcatgtg gcagcagagt gtcccagcaa 960
gtgtctggtg gaggtcctga ttgccataag cagcctcacg tctccgctgc tgttcacagc 1020
ctctggatat ctgtcattca gcatcatgag aatcgtggag atgtttaagg attacccgcc 1080
agccataaaa ccatcctacg atgtgctgct gctgctgctg atgctagtgc tcctgctgca 1140
ggccggcctc aacacgggca ccgccatcca gtgcgtgcgc ttcaaggtca gtgcaaggct 1200
gcagggtgca tcctgggaca cccagaacgg cccgcaggag cgcctggctg gggaggtggc 1260
caggagcccc ctgaaggagt tcgacaagga gaaagcctgg agagccgtcg tggtgcaaat 1320
ggcccagtga cccccagacg cggaaaccgg gtggcagcgc ccagcctggc cccaagcatg 1380
gaaacgcaca acccctaatc gccctgagct actgcttcta acacctcttt tcccttgtgt 1440
gagggcaaac caggctgcag gtggggtttt cacttcctag ggtagtttaa ttttaaaata 1500
ggccaatgtt ggctagtctg tgcctcagtg agatcagtca gctccgagtg gctcccgtgt 1560
cgtaacagca ggagcatggc cgcaacttcc caggccgagg aagggccccc ggctcggcct 1620
cttgagagcc ccacccctga actggcccca gctcctcttc ctgcctctct catggcttgg 1680
gctggagtgg gctctctgga cctgaccaga ctgtgggtcc ctgcgtctcc tgcccactct 1740
gaccgggctt cctccctcca cgcttagggt ctgtcccggg tactcagtca gcccagtggg 1800
atcttaccca cttccctgca aggtgcacct gccccaggct caggctgccc agcggctctt 1860
cctggacagt gagagcaggg cttggcgcct ctgtcctggc ccgggagccg caggggcccc 1920
tcctccagag cctgggcgca agcgacacag gctgccgctg ctctcccagg tgaaatccac 1980
accagtccac gccgggtcgc ctgccctgtc tccctactta gacccagtca ttctagaggg 2040
atccaccgcc acactggccg gcccacgtcc tgggtgctgt catgcccagc ttggagtgcc 2100
acgtggccgc tgcccacgtc ccgggcactg tcatgcccag cttggagtgc cacatggccg 2160
ctgcccacgt cccgggcact gtcatgccca gcttggagtg ccacgtggcc gctgctgtga 2220
caggcagtgt tcttgggggt ggggctgcat ccaaggcttt gtaaaccggc tggaccacgt 2280
ctccctggcc ccagtgaccg gggaagctga gcccctcccc tcctgtgttt gctcccatta 2340
ctcaaaatgc aggacagatc aggtcagagc ccaggaattc tcacaggttc acccagcgcc 2400
ctctacctcc tagcaagtac tttgtcttga tcctcactga gaaggcccca gggcagcggt 2460
cttctccatc tccgctgttt tggggtctta gggtacagcc caggcggtca ctgcccacct 2520
gccaggctgc agggacagtt gggtgtgaga ataacactgg ctttgggtag tgccatggcc 2580
aggagtgggt ttccctgcgt ctcctcgtcc cgagggcgcc tgggtcctcc cagctgacgg 2640
cagtaaatcc acagtgagtt ggggcgactg tgaaactgga atgctgttac tttgataatt 2700
actttccagc aggtgttttc cttcacaatg gttttgtttc tttccttctg atctgagaag 2760
acatgaacgt tttctcttca ccgccgtggg gtgtattgac tggtccccca tgggctgctg 2820
gaaaggcccg gagatgcatc tgtggcctgg ggccatcaag atcaaagaac caggaggcct 2880
gggagatgca gctggatggg gcggcctgca gaccctgcca gggggtttga ggaccctccc 2940
aggtttccca ctgcggaaca ggagtgactc tggctgccaa gataccttca tggtgttcat 3000
gacaagtgga atcattattt tcaaccattg aagggggatg caggcaagac accttcccag 3060
ctgctcctag aggggacaag ccaggccctc tctgcagtcc tcggcagctc cggaaggaca 3120
cagtcagggg ccgggcaaac actttggcca cagccccaaa caagcgccac cgtgggagag 3180
gagaggctgc tgtcactggt accggatgca gaccccaccc tgtctgcagg ccacccccac 3240
ctccctgcag ctttgaggct ggcggggtct gctcctggga atggggtggg agccacaggg 3300

acgacccggg gcgggctgat gtcttcttgg gggcagacca gagagctcaa gtttcagagt 3360
cagaattagg cacttggaac gtttttgctg gcttgcactt tcttattttc ttattttaga 3420
gcgcttaaaa aatccggaaa aatggggttt aaaagaactg tctctttcag tctacatttt 3480
tgtttaatac gcttgagcaa taaacgctga cttgc 3515

<210> 3
<211> 377
<212> PRT
<213> human

<400> 3

<210> 4
<211> 377
<212> PRT
<213> human

<400> 4
Met Thr Gln Glu Pro Phe Arg Glu Glu Leu Ala Tyr Asp Arg Met Pro
  1               5                  10                  15

Thr Leu Glu Arg Gly Arg Gln Asp Pro Ala Ser Tyr Ala Pro Asp Ala
             20                  25                  30

Lys Pro Ser Asp Leu Gln Leu Ser Lys Arg Leu Pro Pro Cys Phe Ser
        35                  40                  45

His Lys Thr Trp Val Phe Ser Val Leu Met Gly Ser Cys Leu Leu Val
    50                  55                  60

Thr Ser Gly Phe Ser Leu Tyr Leu Gly Asn Val Phe Pro Ala Glu Met
65                  70                  75                  80

Asp Tyr Leu Arg Cys Ala Ala Gly Ser Cys Ile Pro Ser Ala Ile Val
                85                  90                  95

Ser Phe Thr Val Ser Arg Arg Asn Ala Asn Val Ile Pro Asn Phe Gln
            100                 105                 110

Ile Leu Phe Val Ser Thr Phe Ala Val Thr Thr Thr Cys Leu Ile Trp
        115                 120                 125

Phe Gly Cys Lys Leu Val Leu Asn Pro Ser Ala Ile Asn Ile Asn Phe
    130                 135                 140

Asn Leu Ile Leu Leu Leu Leu Leu Glu Leu Leu Met Ala Ala Thr Val
145                 150                 155                 160

Ile Ile Ala Ala Arg Ser Ser Glu Glu Asp Cys Lys Lys Lys Lys Gly
                165                 170                 175

Ser Met Ser Asp Ser Ala Asn Ile Leu Asp Glu Val Pro Phe Pro Ala
            180                 185                 190

Arg Val Leu Lys Ser Tyr Ser Val Val Glu Val Ile Ala Gly Ile Ser
        195                 200                 205

Ala Val Leu Gly Gly Ile Ile Ala Leu Asn Val Asp Asp Ser Val Ser
    210                 215                 220

Gly Pro His Leu Ser Val Thr Phe Phe Trp Ile Leu Val Ala Cys Phe
225                 230                 235                 240

Pro Ser Ala Ile Ala Ser His Val Ala Ala Glu Cys Pro Ser Lys Cys
                245                 250                 255

Leu Val Glu Val Leu Ile Ala Ile Ser Ser Leu Thr Ser Pro Leu Leu
            260                 265                 270

Phe Thr Ala Ser Gly Tyr Leu Ser Phe Ser Ile Met Arg Ile Val Glu
        275                 280                 285

Met Phe Lys Asp Tyr Pro Pro Ala Ile Lys Pro Ser Tyr Asp Val Leu
    290                 295                 300

Leu Leu Leu Leu Met Leu Val Leu Leu Leu Gln Ala Gly Leu Asn Thr
305                 310                 315                 320

Gly Thr Ala Ile Gln Cys Val Arg Phe Lys Val Ser Ala Arg Leu Gln
                325                 330                 335

Gly Ala Ser Trp Asp Thr Gln Asn Gly Pro Gln Glu Arg Leu Ala Gly
            340                 345                 350

Glu Val Ala Arg Ser Pro Leu Lys Glu Phe Asp Lys Glu Lys Ala Trp
        355                 360                 365

Arg Ala Val Val Val Gln Met Ala Gln
    370                 375

<210> 5
<211> 34634
<212> DNA
<213> human

<400> 5
catggggccc ccttacaagc atcaggacca catcacatgc aggtgggatt ctgatacatg 60
attaaggaat tttccaaatc agcagggaaa aagtgagcta ctcagatgac tggctagcca 120
ttaggaaagc aatgtctggt tccttctctc agtctgtgca cccaaataaa ttccaggcag 180
gtcaaatatt taaatatcaa caaacaggcc aggcatggtg gctcatgcct gtaatcccaa 240
cactttggga ggccgaggtg gaaggaccac tcgaggccag gagtttcaga tcagcctacg 300
caacacagtg agacgccatt tctacaaaaa aattttaaaa attagtcagg tatgatggtg 360
tacacctgtg gtcccagtta ctcaggaggc tgaggtggaa gaatcgcttg agctcaggag 420
ttccaggctg cagtgaacta taatcgcact actatactcc agcctgggcg acagcgcaaa 480
gctctgtctc aaaaattgaa aaaaaaattc taattgtcaa taaacaattt tcagagtaaa 540
atttgaatat taggaataag aagtggaata agcctcccta aggcttctta cagcatccaa 600
aagctacatg gaaaagaggc acacttaact acaaaaaatg aaactataaa acacagtcaa 660
agatgatcct ggggaaaata ttcctaacac tttcatataa taattttcct taaaaagctc 720
tgatttccct aatatataag ctttaacaaa agaaaaagct gaactcaaca aaaatgtgga 780
caaaggaccc aaaaataatt catagaaaaa acatgaggcc gggcgcggtg gctcacacct 840
gtaatcctag cactttggga ggctgaggtg ggcagatcac gaggtcagga gctccagacc 900
atcctggcta acacggtgaa accccgtctc tactaaaaat acaaaaaaat taactgggtg 960
tggtggtggg tgcctgtagt cccagctact caggaggctg aggcaggaga atggcgtgaa 1020
cccaggaggc ggagcttgca gtgagctgag attgcgtcac tgcactccag cctgggcgac 1080
agagactccg tctcagaaaa aaacaaaaac aaaaacaaaa agaaaaagaa aagttaccat 1140
acttaaggct taagtgcaaa ttaagtgcag tctctcattc tgctcctaag agtataaatt 1200
tgtatattat tgatgttact ttggtgataa ccattaaatt gttgaaaaag caagaaaata 1260
atggatatat aattgctatg ttctccacag cataaattca taggcaaact ttactttcta 1320

aagtatacat gtctatgttg tttgaatgat ttacaaagac tgctgtttgt ttatttattg 1380
agacagggtc ttgctctgtc atccaggctg gagtgtggta gtgcaatcat aactcattgc 1440
agccttgacc ctctgggctc aagcgatcct cccgcctcag cctcccatgt agctgggact 1500
acaggtatgt gccacgacac caggctaact tttgtgggtt ttttttttgt agacacgagg 1560
tttcaccatg ctgcccaggc tggtctctta acacctgagc tcaagtgatc tgcctgtctc 1620
agccttccaa agtgctggga ttacaggcgt gagccactgt acccagtcta ttttttaaaa 1680
cataaatcat aaagagcaaa agcatcccct tgaaggtaca ttccactctt tctcccttgg 1740
ccgggtcatg tctgatagat taaacacaag gaaaaacaac acaaagtccc gtggcgaaga 1800
aaaactgttc ccaaatgaac tcaaacctta ccctgattgc tttcagtcca ttggacactt 1860
tattttcttc cacaactgca atcacttcct gtccaacatt cagaagcact ctgctagtca 1920
cagcatcact ggagaagtag atcatatcat caatcatgcc ataatcgctg cagtaccttg 1980
tcacgacacc ccgtacagtt ttcagcttag tgtcacctga ggtaggtaaa caaagaagtt 2040
atcttggcca taaaagcaag cgtacgctac agtgaacccc accagagaaa ggcagagtgc 2100
aaaagcctcc tttttttgca gttcggaggc actgactccg aacgggctga cttgagacag 2160
agagaatccc atcagagagt gctgtgtctg gaacgtgggg tttggaggca gacagcccag 2220
tttcctcaga agggcacagg tagtggttgt ggaggtgaca gccctgactc cacaacagac 2280
tgcatggggt tgactcctag ctcttctact gccattggtg taaccctgga ctaggtcctt 2340
aactactctg ggcctgttgc catctgtaag atggagacta atcgtcacta cccaagagag 2400
ctgctgaagg gttaagtggc agaacacacg tgcatgtatg gacctctgcg tggctgacac 2460
gcgttaagtg ctatgtaaat ggccacttat atcagcatct gggagtcatt agcatgcacg 2520
gttttattag tactatcgat tatcttttta ttaccatcag cagcagcctc ggaagactgg 2580
agaatctcaa caaacacggt gctggaaaag gttcggggct gggcagacaa gtctggccca 2640
ggagcagcgc acgccctcag ccgtctggcc actgctgcag ccacggtcct gagtcctgaa 2700
cgtgccttta ctgcagcttc ccagccacgg gaggaacctg gggcgacggt tacagtacag 2760
gcataggatc tgagcgctgc caagagctgg agctccagct accgctgagc gcgtgttcta 2820
ccgaagagat ccacggagca agtgctccag gtaacacagc cggccccccg ggcatcggcc 2880
ctcgtggatc cagaggccct gaacacgctg cctgcagcag gaagctcccc tcctgaagcc 2940
tgttcctgtc ttttctcaca gaaggcaatt agggacccac tactgtggat tccattccca 3000
cataaggaaa catcgggaga agccatcaga acctcgggag aggtcgggcg tggtggctca 3060
agcctgtaat cccagcactt tgggaggcca aggtgggtgg gtcacctgag atcaggagtt 3120
cgagaccagc ctgaccaaca tggcgaaccc cccctcgact aaaaatacaa aaattagccg 3180
ggcgtggtgg cgggtgccta taatccccgc tgctcgggag gctgaggcag gagaatcact 3240
tgaacccaga aggcggaggt tgcggtgagc cgagattgcg ccactgcact ccagcctggg 3300
caacaagagc gaaactccgt ctcacacaca cacacacaca aacacctcag gagaaaggaa 3360
cggtgagggc atccgggcgc atcccgggct ttagaaagat gggcttgaaa accagacaag 3420
gaaatggcac gccttggcgc agcgcgttct gaaaggacag gccaagtctg cggggccgcg 3480
tttttcgggg ctttctagac acaccaccgc caatgacgct gcggacaggg aggaacgaca 3540
tggagagacg acttggcagc tgctcggcgt aaacacacaa agtgagggcg ggaagagggg 3600
ctggtgtcac cggagagacc tcacagagga cgggcgccca gagcggcggg atgctgtggg 3660
gtcggaagcc tcctgaggcg aacggggatc cccgccagcg gagatgagga aaggccgggc 3720
ccagcgttgc ctgccggccc gcactgcact caaagaccta tgggcgcggc tccctcgtgg 3780
ccgacacgcg agggcccggg acccgcctcc cagccgcctc ccgcgagcca ccttccgcga 3840
gctcgggctc cagctgcccg gcttcctccc taggggtgtc cgccgtcctc cagaagaagg 3900
ccaccagctt ggctgcgagg ctcagcatgg ccctcgcccg gcctaggctg ccgtcaccgc 3960
cgctgccgcc gcccgcacgc gcccgcgccg ctcccgccgc ccgccaccaa tggggtcgcc 4020
gcgcctgggc atgcgccttg gggtcgttgc gcgagccggg acccgtagcg gtcagaggtc 4080
ggcggttggc gcctggcagc tcctgacaag gcgaggtgcc tggtgggagt gacgtcatgc 4140
cgagcgcgca aggcggtgtg accgccgggt gggcggggct tcgagcgggc gggcggggcc 4200

tcgcgtgggt gggcggagct tcgagcgggt ccgccttctt gccgcccaca cattagggaa 4260
aagtgcgctt tcaccaccca gagtgatgtt aggagtcgtt ttttgttggt gttttttatc 4320
aagttgagga agttccctct attcctgttt ttccaagagt tcttatgagt gggtgtgaaa 4380
ttttctgcaa cgcttttctg catgtattca aatgatcctg tagcttttta tttcagccca 4440
tagggaatta cattgtttgg ctctccttgg aaaactcact tggtcacgat tcgttatcct 4500
tttggtgtat tgcctgactc agcttggtaa cgctttgtcc aaggttgggg acggtgctgg 4560
tgaagcagat tgctctgtgg ccgacctccc gttttctgtg tctgtctggt ttggggtcgg 4620
tttaagccgg gcctgcagtg acctgggagt aggatggggt aacttattcc ctaaaaattg 4680
tggtagtatt cgctggggaa cccatttggg cttcgagatt tctttgtggg aaggttttta 4740
atgagaaatt cacttctgca tcagctagag ggctattcaa gttatcaagg taatattgaa 4800
taaactttgg taattctttt aaggaatttg tccatttcat ccaattatta gatgtcagat 4860
tattggcata aagttgttca gcatatttcc ttattatcct tttaacgtct gtatggtcca 4920
taatgatgag ccatctttca ttcttgacat aggtaattta tctcatccct ctcattttcc 4980
ttgatcagtc tatatttatc aattttattg agtttttaaa aaaaatcatc ccttgatttc 5040
attgattttt ctctatttta aaaatatctt tacactttgc gaggccaagg tgagcagatc 5100
acttgaggtc aggagttcga gaccagcctg gtcaacctgc caaaatcctg tctctacaaa 5160
aaaatacaaa aaaaattagc caggcatggt ggcgcatgcc tgtaatccca actacttggg 5220
aggctgaagc agaagaatca cttgaaccca ggaggtggag gttgcagtga gctgagatca 5280
caccactaca ctccagcctg ggagacagag caagactctg cctcaaaaca aaaacaaaaa 5340
caaaatacgt atatatatct ttactattcc agtggtttct gtttttattt atttcttttt 5400
tttttttttt gagatggagt cctgctccgt cgcccaggct ggaatgcagt ggcatgatct 5460
tagctcactg tagcctccac ctcccgggtt caagtgattc tcctgcctca gcctccagag 5520
cagctgggat tacaggcgtg cgccaccacg ctggcttttt tttttttttt tttttttttt 5580
tttttttgta tttttagtag cgacaaggtt ttaccatgtt ggtcaggctg gtctcgaact 5640
tctgacccgc aggtgatcca cccgccttgg cctcccgagt gctgggatta caggtgtgag 5700
ccactgcgcc cagccccgac ttatttttga ttgacatagc tttattttcc taggtcagat 5760
ctgtcagtct gatgacagcc tggaccttgc ctgtccatgg atgcagagtg tactgatagt 5820
tcttgctgtg ggaagggaga gagtggcgca ctccctagca tttcaaatcc ccgctaagcc 5880
tgtgtcttgg gctgggtgct ggaacacata caggagccag cagggtccca gggacctact 5940
ggttggtggg agattagctt ggaaacaaga gcgagctgca tgagggctgg tggctcatgg 6000
gctccctgcc agggcagctc gcagcgggtg ctgtgcctgc agggctgccc ctcagctccc 6060
cagctcgggc cactccctgg gattctggac tcagtagacc cggcagatgc ccacagacat 6120
ctcggcctca tcatgtccag aatggaactc cagatcctcg ctccacacct gctccaccct 6180
ggtcttctca tctctgtcct cactgctcag acagaactca gcaacgtcgc cctgacagca 6240
gattgcgtcc gctcttcaaa ccctgttcag agcccagtcc ctcctcacca cttgcccact 6300
gccacctcaa tgccaggcag cacctgctcc tcccaccccc cagcctgctc tccacccagt 6360
gagtgaccgg atgctgctct gcccagaccc ctccagcagg tctgtctcag agttgaagca 6420
gagtcgtctc catacccaca ggtgctggca cctggctgtc ctacctgtat cccccttgca 6480
tggtcctcgc ctgccttccc caacctgtgg cctttgcttc aactctgcca gcctcccctc 6540
cctgcctcct tctcactgag gcctgccctg gcccctgcaa acagcagccc ctcatttctc 6600
tcccctgact tcttttccct catagaggaa atcatgtgtg tcactggatg cattagtctg 6660
ttctcacgct gctataagga catacccaag actgggtaat tttagagtca agtccttata 6720
aaggaaagag ctttaattga ctcacagttc cacagggctg aggaggcctc aggaaactta 6780
caatcatggt ggaaggggaa gcaaacatgt ccttcttcac aaggtggcag gaaggagaag 6840
tcttgagcaa aagcggggaa agccccttat aaaagaccat cagatctcat aagacctcac 6900
tcattgtcag gagaacagca gcatgggggt aaccaccccc atgattcaat tacctcccac 6960
cgggcccctt ccacgacatg taggaattat gggaattaca attcaagatg agatttgagt 7020
ggggacacag ccaaaccata tcactgtata acacactaat ttgtttatcc tgtgcactcc 7080

tgtgccccca atgaatgtaa acagcctata ggaaggacgt cacttctttg tccaccccat 7140
aaatgtttgt tgaatgttga atgatgatgg gacatcagca ggcagaggga tgaagggcat 7200
tttaggagca agaatttgtt ggaagattgt atgaaagaac agggcctgtt ctagaaacta 7260
gtgttgccaa cagaaacttg ggagcatttc tcaagtgatg acttatgatc tgggaagctg 7320
agggatttgc tctgcaacct ctggttcagg gcagcttgtt tgtatttcta agtggagagc 7380
aacttttgaa agaaaaaaat gttgaaatcc gattaacatt tttttccaaa caaaaagaaa 7440
cacaggagga ttcgatccag ttggacccac tcccactaat cccgtttatc gctttaaaag 7500
atagtgaagt ttgggaggtt tctcaatcca ttaaaatgag ttcttgactt cacagattat 7560
agtttttgaa aattatttct catccttgct tgattgaaat gaagacaaca gaacattggc 7620
tgtggtggct ggcacaaaat taactaaccc taaaatacta gtatagctta aactttcgtt 7680
tattgctaaa aatttatcac tgctgtttcc tgtgggggtg aggttgagca aagtgttttg 7740
agctgcattt atgcgtactt gacacttacg cattttgatc gaggtgattt agtgggcatt 7800
ttcactggga cagggatgct tgtatgtgta atcttactaa aagctaataa aaacttacta 7860
aaagctaata aaagcttact aaaagcttct tgcttgattg aaacgaagac aacagaacat 7920
cccatggtct ggaacctgat gactttgctc aagttttaat gtgggttcat ggtttaagga 7980
gctggttttt cagaaacttt agtttgagcc tttttacaat gtgcacaaag aacccgttgc 8040
tgtagttgtc agggtgccag tgtctctggg cgacacacat tactgtggtt tttctctgct 8100
tggtgagcag agataaaggg ggcagcagga ccgggcccac cagccatccg ggctgcccac 8160
gcaaaccaca gggccgaatc cggagccgcc caaggccaca cagctaagcc gagtgcgtga 8220
atgcttatgt gaccgtgtga aggaggttcc caccgtgtgg ctgtggggga tggaaaaagg 8280
ctacttggaa agatgtagaa gaccttcgag taaacagtta cgtttcagaa acagagcctg 8340
ctcagaatgt gtacttggtg ggattctatt cttagggacg cttctttctt ctgagagacc 8400
cgagctctgt ggcgagtggc acaggcaggg ccccttcctt tcctagttgg gttctgacag 8460
ctccgaggca gtggtttaca caaccaacac gaaacatttc tacgatccac ccgattcctc 8520
ccctcattga tattcaggaa gcagctctcc ttcccctgcc ttcagctcaa gtttgctgag 8580
cttttgtttc atttgtgaat acttcttgct ggaagtccct cacccagaga ccagtgctcc 8640
caacggcaga gcagcggggg aggtaagtgc tcagacatta agccgttgag tagaggcatg 8700
ttttgcaatc tctcgtttag ctaccaattg gagcagttta acgtgcttga gtccccggcg 8760
tgatgtgttc atggacgaag ttgccgatgg atgttgtcag gtgttgcacc tggagggccc 8820
agatggatca atgaggcatt taaagtgtag aatctcaccc tgaacccaga ctattcaggt 8880
ccgtttttct aggaaagtta tttctttttc acttttagat tcaagtgctc attttcttct 8940
tcaaatttag agcacttcag tatgaacaat atttcttttg ttaaaaaata tttcttggaa 9000
acaacttatt gaagtttcct cagagtggcc aaagccgatg ataattttat tccagataaa 9060
gaactggtga cacgtggctg tacattcagc acagctgtgg tgtccccaag tgccatgacc 9120
caggagccat tcagagagga gctggcctat gaccggatgc ccacgctgga gcggggccgg 9180
caagaccccg ccagctatgc cccagacgcg aagccgagcg acctgcagct gtcgaagaga 9240
ctgcccccct gcttcagcca caagacgtgg gtcttctctg tgctgatggg ggtgagtagc 9300
tccacactaa gcccttggag ggccacgagc atctggtccc tctggtgacg gactgcagcg 9360
ccctggagcc ccaggtggga cagagagagc aactctcaga ctaaccctgc tgctgcagaa 9420
ctcttggatg ccgacgagct tgttttgctt gtgttttcac aattattttt attaatttat 9480
gggtgtctgt tgtgccagcc ttgagctgct ccaggttgtg gcttagtccc accagggtag 9540
ggacagtccc agggccatga atgggcccca ggagtctcca tgaccctcgg ctctgggggc 9600
ccagggtcct ctgaggggtc acctgcaaag gtgggaggag gcgtctcagg ccttcaggtc 9660
ggccctcaca gagagaacct cagggacctc ctcctcctag ggagggcctc aggggccggc 9720
tgggagggag ctggggaaca aaggaacagg tctggggtgg acgggcagcg ccagatgtcc 9780
gagggatttc cctggggtcc tggccattcc ctgcacagtc gtgggcacag agatgattgg 9840
aggagccact tgggcctgag tgtgtgcagc tgctcagagc catgctgcag gacagg[?]ctc 9900
tatgcaggga cccccggttc cgtgtagctt gggctgagaa tgagatccag gctgtgattt 9960

ctcccctccc tgtgccctgg cctccgccgg cagcccctgc agcacccaca gtggtcctgc 10020
ccacctcccg tggcctgagc atgccccacc aagacaggcg tctgcctcgg agctggggac 10080
agaggcggct cccgggtggt ggtggccatc tccgggctct gccctcctcc acctggtgcc 10140
ctggacttgg tcgcagcccc tcccccgccc aggccagatc ccctctgccc cagacctcgc 10200
ttcctctgcc aggaccctgg gtcccagtga cctttgatac tgactcccct cagactcacc 10260
ccgggggtgc tgtgcgtgag agagagcccc ctccccaccg cacccctgca gtgctgtgcg 10320
tgagagagag caccctccct gcctcctgct agggtcttcc ctgctcagga gcccctgggc 10380
ctggagcccc tgcctggagg gatcttcttc cagatctgga aattaggttc tgcagacgga 10440
gggggatggg cagttgtttt ccagtctcgt gtctggacct gggtgtggga tctggaagac 10500
tgcacgcaca acatgttggg gcaggagtat tttgtgggga gaggtgccat tccttgctct 10560
aaatattggc agatcccctt tcttctctcc tccagataac agcgtggggg gtccatgctc 10620
cgacatggct gggggaggct ggtccatgtc ctgggctgcg gcggggctgc gtctgtccta 10680
caggaggagt ccaggatggg cgggtcccag gtgcgggtgt ggttgatgct gggggacgtg 10740
ggtgagtcac caatacacag acagtgaagc agaagttgaa gggtcagggt ccagcctggg 10800
gacggggcgc gttgtaacct gggattctgt cgctgtcttg cagagctgcc tcctggtgac 10860
ctcggggttt tcgctgtacc tggggaacgt gttcccggct gagatggatt acttgcgctg 10920
tgctgcaggc tctgtaagca gcttcctcca cgcctctgcc agtgcccaag ctcgccggca 10980
cgtgctctgg tttctttgtt ctgagatgtg caggtttctg tcacctgtac ctcattcaaa 11040
cctcttcata tgggccgggc acagtggctc atggctgtca tgccagcact ttgaggggtg 11100
gaggtgggtg gatcacctga ggtcaggtgt ttgagaccag tctgaccaac atggacaaac 11160
cccatctcta ctaaaaatac aaaaattagc tgggcgcggt ggtgggtgcc tgtactccca 11220
gctactcagg aggctgaggc aggagaatcg cttgaacctg ggaggcggag gttgcagtga 11280
gcccagattg tgccactgca ctccagcctg ggcaatgaga gtgaaacttc gtctcaaaac 11340
aacaacaaca agaaaaaaac aaaaaactct tcatatgact ttagtttcac gtagagttca 11400
tcacctttga gaattactga tgaagcaggc ctgaaccttg ctctgcagac gcctgtgcag 11460
ggaattgcgt gttgattctg agcctgcggc tgcgtcctgc cagagctggc agcatctgcc 11520
ctcaggggac tgggacctgt gccccacatc ctcccaaagc ttcatgttgt gtttttagaa 11580
acttacacag tgccaaggcc ggtggaggca cccactgatc ctcagagcaa gggaggacag 11640
acaaccgctg tcccctggtc ctctgtgaac cccggccact gcctccccag cgtcccggca 11700
gctagagcac catgctggct gacccctggg ctctggcccg caagccatgg accccatgcc 11760
cctctggccc gcaagccgcg caccccatgc ccctctggcc cgcaagccgc ggaccccatg 11820
cccctctggc ccgcaagccg cggaccccac gcccctcctg cttcagctgg tcccgagctc 11880
tcccctcctc tcgcttccac atgcctgctg ggtctctggc ccctgccctt tctctaggga 11940
cactgttggt gactgtcccc agcagcgccc ccgaaatgtc ttaaaaacaa agtggatgcc 12000
ccgccccgtg ctgtcctggg tctctgctcc cttagaacaa agccctttgg ggcaggtgac 12060
ccgtcccact ctgctgccct ctctggaagc ttctccgtgg cctttatcct caccatggga 12120
gccgcccttg tcccagtgga gctgtgagct gtgccccttc agcctcagcg gctgccggat 12180
atgggcaagt gcccaccccc gcccagcctt ggccccactc ggccctgccc ccggcctttc 12240
ttctctaccc tgggccctcg tggcacctga gtgggtgctg agctgagcct ggctgtcctg 12300
gccctagctc tgcccctcgc tcaggccccg acggggcttc ttctgcctcc tcacccagca 12360
ctgctgtgct cagcccctca gggcctgcac ccccgactca gggcccgggg tcccatggct 12420
gcaggagcaa ggccctgcac ggcaagggcc acaggcctgg gctggcccga ctccatccac 12480
ctccctgtcc ctgacgtccc aacctccctc cctggccctg gactcctagc cttggtgtgg 12540
ccctgtgggg acaagaaggc tgaggacaag aaggctgtgg gactcctttc tgcctccctc 12600
acatgcagag tcttgctact tcctttgctc cttccttcct ctttctttct ttcttttttt 12660
ctttctttct ttctttgacg gagtcttgct ctgtcacaca ggctggagtg caatggcgcc 12720
gtctctgctc actgcaacct ccacctcctg ggttcaagca attctcctgc ctcagcctcc 12780
tgagtagctg ggactacagg cacccgccac cacgcccagc taattttttt tgtattttta 12840

gtagagatgg ggtttcacta tgttggccag gctggtctca aactcctgac tctgatccac 12900
ccgcctcagc ctcccaaagt actgggatta caggtgtgag ccactgcacc agacctcctc 12960
tttctttctt tctttctttc tttctttctt tctttctttc tttctttttt tgagacaagg 13020
tctcactcta tcaccgagta ttcacagctc actgcagcct cgaactcctg ggctcaagag 13080
atcctcccag ctcagcctcc ggagtagttg ggactagagg tgtgagccac tgcacccggc 13140
tgatactttt tccttttttg ggtaattttc agcaaagacc aggtcttgtg cctgcgtgag 13200
ttacattcta ggcttcaaag cttatccagc tagaagagcc ttcattaaca aatatctttt 13260
tagcacatca gaaaaataaa aacttctttc agaataaatt attttgaagt acctgggggt 13320
tgggcgcagc ggctcatgcc tgtaatcgca gggctttggg aagctgaggt gaacagatca 13380
tttgagccca aagttaagat cagcttgggc aacagagtga gaccccatct caagaaaaaa 13440
ttaaaactga gaagtacctg ggggccttct agtcaagcca acccactgtg ttgcatggac 13500
tggcaacact gttttgtgaa tatgattcag atccttctgg aagcgcaaat gttagagtat 13560
ttcagagtct cagttgctca ggtgagcggc aggctcatgg gattccggtt tcattccagt 13620
gcatcccctc ggcaattgtg agcttcaccg tctccaggag gaacgccaat gtggtgagtc 13680
tagcttgcag gcagcctctg ccagggagaa agccggaggg gctgacagac agtgtgccca 13740
tgtgtgcttg tgtgtgtgcc cacgcacaga cgttgtggca ccgagagggg cccccgaggt 13800
tgccccccag acagtaaagc tcctgcccag cccagccttt gccgcacaga ggggagagcg 13860
ggctgagcac gcaggactcc ccaccattat gtcctaatca ggataatttt tagtgaatgg 13920
cctgaagtgt ggtttatctg ttaccttttt tcttttatta ttcaaagaag gaaaacccct 13980
cctgccttgt ttttcagatt cccaactttc agatattgtt tgtttccacg tttgctgtga 14040
ccactacgtg tttaatttgg tttggatgca aactagtcct gaacccatca gcaataaacg 14100
tgagttcaca cgagcttcgc gcagacgcct gacacccaca gccccactcc cacgaatggt 14160
gtcagcaaat gtttgttgag ctggaaatag gtggtaaatg ttattcacag ctcgtgagtt 14220
tgactaaagt tgagagattt atgcatccaa agaaaataat tgtctttatt gaagtctcgt 14280
gattctgcgg gacagattta ttggtaaaca atgtcttttc caacagtcct taaaaattaa 14340
cagtgacata atactatgtt tcatccaatc tgtgtaagac agttgttcaa tgcacctcta 14400
aacaaaaatg tgaaattgta gggggcagag gttgtgggag ccgagatggc gccactgcac 14460
tccagcctgg aagacagagc aagactccgt ctcaaaaaaa aaaaaaaaaa aaaaaagaca 14520
aaaatcccaa aggaaaccag gacctcatcc gagatactgc aaggagggcc tcctgctttg 14580
tgtgacatgt ggaatgcact tgatgattgg ggaaagctgg aggaggcttg agtatgtcct 14640
ggccactctc gttgggcagg aaaaatgagc agacacagga ggacccagtg ggaacaccca 14700
cactgccttt ttgttttgtt ttgttttgtt tttgagaagg aatctcgctc tgtcgcccag 14760
gctggagtgt agtggcgcca tctcggctca ctgcaacctc tacctcccgg gttcaagcaa 14820
ttctcctgcc tcagcctctg gagtagctgg gattacaggc gtgtgccacc acactcggct 14880
aatttttgta tttttagtac agacaaggtt tcaccatgtt ggccaggctg gccttgtact 14940
cctgacctca ggtgatccat ccacctcggc ctcccatagt gctgggatta caggtatgag 15000
ccaccgcgcc cggcctgtga cgcttcttta ttaagacgga gagcagatgg ggtgtggtcc 15060
ctgggtcagt ggcttgtatc tgaactgctc agaggcaagt tactcactgt agctgtcggc 15120
tggccctggg aggagctgtc tcttcaggat caggaaggag ccagaggtca aagcaccaga 15180
aatacagaaa ataaaagaca aggttaatgt cacacttttc tgaattttag agttctcctt 15240
atgcttttgt ctttttgatc tgtcattttt tttttttttt gagacagtct cgctctgtcg 15300
cccagactgg agtgcagtgg ctccatctcg gctcacggca acctccgcct ctcgggttca 15360
agcaattctc ctgtctcagc ctcccgagta gctgggatta caggcgtgcg ccaccgtacc 15420
cagctgattt ttgtattttt agtagacaag gggtttcacc atgttggtca ggctggtctg 15480
gaactcctga cctcaggtga tccaaccgcc tcggtccccc aaagtgctgg gatcacaggc 15540
gtgagccgcc tccctctgcc tgatctgtca gtttctaaga aaagtgtttc aagtccttcc 15600
ccacctctac tgatatatct acatcttcct gtggctctgt tatgacatca tatatttcga 15660
ggctctgttt ttagttgaat gtggttctgc actctttctt ttttaaatgt attttaaaaa 15720

ttgtcaggcc gggcgcggtg gctcacgcct gtaatctcag cactttggga ggccgaggtg 15780
ggcggaacac ctgaggtcag gagttcgaga ccagcctgac caacatggag aaacccctct 15840
ctactaaaaa tacaaaatta gccgggcgtg ctggtaggtg cctgtaaccc cagcttactc 15900
gggaggctga ggcaggacaa tcatttgaac ccaggaggca gaggttgcgg tgagccgaga 15960
ttgcgccatt tgctctccag cctgggcaac aagagcgaac ctccgtctca aaaaaaaaaa 16020
aaaaattgtc agacagctac actgactttt taaaatgtat gcttctatga atattaacac 16080
acatataaat tcatgtcact gccaatacgg tcagggcctg cagtcgtttc attaccctcc 16140
aaaaaacctt atgttctcag tgcttgacaa tcatgcatga agctactcta aacattccgt 16200
gaaggctttt gtgggaacaa ggagcgttca gaatggggtc ggtagaccag agcccaaggc 16260
caaatccagc cctggcctgt gtttgtacag cctttgcact aagaatggtt ttaaaaacta 16320
aaagacccca aaggcccatg tgaggcagga gagtgtgcag cccacagtgc ctcacagctg 16380
gccctggatg ggaaaagtgg ggaccgtggc tgagagtcct gcgcagcctg cccgtgaggt 16440
ggtccggtgg acgctgaagc gcggtggcgt gagaaaggcg ggcccggtgc tgaagcgtgt 16500
gtgccccttg gcagatcaac ttcaacctca tcctgctgct cctgctggag ctgctcatgg 16560
cggccacggt gatcatcgct gcacggtcca gcgaggagga ctgcaagaaa aagaaggtgg 16620
gtgcagcttc tgaccccgca gggcaggggg tgcagaggca tcaccccagg gtgagcgagg 16680
tgggtctcag cgtcaccctc ggagggccga tctgggctgc tccagggcat tcccatgaga 16740
cccctctctc ctgggtctca gaaaccccgg ccaggagggg ctctggagct gggtgagggg 16800
ttggcggccg ctggctacag ggaggtgaag acagatgctg taccctcccc tccccctaag 16860
cctgactcag tccagtgctc gtccccaggg gatcaccagg agacaccaca tctgctaatt 16920
ttcatatgca aggacaatgt gtgtgaacat ttcctcaagc ccaaagatcc acgggcgcct 16980
ccaaggcggt ctcgcatcta cacggcaggc tgacctgacc cactgcccac ggcggccgga 17040
tgccagcggc agtgctgagt ccctgtgccc gtgcacccgg ggtgacagtt ctgtttcttc 17100
gtagggctcc atgtctgaca gcgccaacat tctggacgaa gtgccatttc ctgctcgggt 17160
cctgaaatct tactcagtaa gaaaacttcc cttctttctc ttttctctca tactctgtcg 17220
ggggtgactg aggctggatt aaacgtcacg tttcgattcc gagttggcga agagttggct 17280
gtctacaccg catggcggcg tgcgcctcat ctgtggagac tctagtctgt tattggtatc 17340
attggtaata accttcctta tcgcgaaagg tgctgctggt gtcctgtaat ttttaggggg 17400
agtttgttac tgccgtaggt gagttgacac tgttgtcccc gaggaatttt tttttttttt 17460
ttgagacgga gtctcactcc atcagccagg ctggggtgca gtggtgctat ctcagctcac 17520
cgccacctct gcctcccagg ttcaagtaat tctcctgcct cagcctcctg aggagctggg 17580
actacaggca catgccacca cgcccggcta attattttat ttttagtaga ggcagggttt 17640
cggcatgttg gccaggctgg tctcgaactc ctgacctcaa gtgatccacc cacccaggcc 17700
ttccaaagtg ctgggattac aggtgtgagc caccgcacct ggcctctttc tccttcttac 17760
tttcaacttt tcttgccttg tactttggta taattgttat aagcagcacg taactgtatt 17820
ttgttcttta tcttgtcatt ctctctccct agctttttga atgtagccca tttcctctga 17880
gagttattac tgataaattt ctacttattt ttaccatctt ttgagttttc cattttccct 17940
gattttgcct ttttccctcc ttccctgcct tcttttgaat tggcagaggc tccgtccttc 18000
cttttccctc taattttgga tgggaaattt ttcagtattc ttttgggggt tgcccttcat 18060
attctaatac gctccttgat aaacagacca tgcggggtct gtcccccttc cctccgcctt 18120
ggccttgttg tctagcttgt gacatttagt gacattttgc ctttttactc caacaatggg 18180
acagcctcct gtgtgcttga ctgtgtctgt gtgtgtttcc cagtgccttc acttcccctt 18240
atttagcatt tcagtcctct cttaggttcc atttccgttc tgcttctttt ttttttttcc 18300
tttttttttg agatggagtc ctgctgttgc ccaggctaga gtgcagtggt gtgatcttgg 18360
ttcactgcaa cctctgcctc ctgagttgaa gcgattctcc cgcctcagcc tcccgagtag 18420
ctgggattac aggtgcccgc caccacgccc agctaatttt tgttattttt agtagagacg 18480
gggtttacca tgttggccag gctgtactca aactcctgac ctcacgatcc gtctgcctcg 18540
gcctcccaaa gtgctaggat tacaggcatg agccgccgtg cctggctggg aattcagctt 18600

tatttgtgca tctggagttt tgtgccttct ggacgcaagg cctggctgcc caggtccctt 18660
ccggccccca agcccttgct ggcccactcc ttatgcctgg ctttcctgct ggccacagct 18720
ctgtgtttgc gactcacaga gatccttcac gggagcaggt tttcgggcgt gtgtttcttc 18780
cccagccctc cctgagtctg gcggtgctgg ttgcggcccc tccctgagtc tggcggtgct 18840
ggttgcggcc cctccctgag tctggcggtg ctggttgcgg cccctccctg agtctggcgg 18900
tgctggttgc ggccctgatg gtgccgggca gatgagtcca caaccagggc ttaggccagc 18960
agggttcttg gctttgtcca ggaaagaatt caagggcgag ctggaggtga cagacagcag 19020
ctttgtttgc agtggcagta tagcagggct ctccaaccgg caggtgcctc gagcagcagc 19080
tcagagacag ctctgcctcc tatttatacc cacttttaag tatatgctaa ttaaggggca 19140
gattatgcag aaatttctag aaaaggggtg ctaacgtcca ggtggttgga tcattgccac 19200
ggaaaggggt ggtgacgtcc agtgttgccg tggcaatggc aaacccacat ggcacgggtg 19260
ggtgtgtcct atgggaagct acttctgccc caggcctgtt ttcccgagtc ccttcgatgg 19320
ggtccagggt ctgagcaccg ccgcctgcca cactgtgaga cagggaaggg agtctgaggg 19380
tcttggtttt ctgccctcac cactgtcagg tcaataccct cagtttctgt ggacaaaagc 19440
agcagtgggg gaaacacctc tttggctatt ttactccagg agggacgtga aaagccgcag 19500
ctgagggcag agggggtctg ggccaggtga ggctgggggt ccatccaggt gggctcctcc 19560
acggttagat ctggggccag cccactggac cctgctcctg ggggtccatg tctgcacagt 19620
gcattctggg aatctggggg tgtctccact tccttatgag ccgatgctgt cctgtcccct 19680
ccaggtcgtc gaggtaatcg caggcatctc tgccgtcctc ggggggatca ttgccctgaa 19740
cgtggatgac tcagtttcag gcccacacct ctcagtgacg ttcttttgga tcctagtggc 19800
cgtgagtaac ccctctggcc cgcccgccgc cgctctgggc cacactgctc agatcctgcg 19860
tggctgggcc ccgagctcag tcttggtgca ttcagacaga gagtcaccac agagggtcct 19920
cgctggcccc caacttcaga agaactgggg agttaaggat taaatgagtc aaagtattaa 19980
ctgcttttat attagtttgt gtgattattt tatattattt ttcctgaaaa aaataccaat 20040
tatttttatt attctaatct ttttaacata tgaatgtcag gctgtagatg tttattattc 20100
tatataattt tctaatatat aatgcattta tattacacta tgtgtttcat acaatgcagc 20160
atatttctgt tattacttta cattaaatgt tactgcagca gatgtgtcac acagcacctg 20220
acacatgcca catgctcaat acatgggcat tgttttattt attttattta tttatttttt 20280
tgagatggag tctcactctg ccacccaggc tggagtgcag tggctcaata tcacctcact 20340
acaacctctg cccctccccg ggttcaagta atttgcccac ctcagcctcc caagtagctg 20400
ggattacagg cgtggaccac catgcccagc taattttttt gtatttttag tagagaccgg 20460
gttttgccat gttggccagg ctggtcttga actcctgacc tcaagtgatc tgcctgcctc 20520
ggcccccaaa agtgctggga ttacaggtgt gttccctcag aacagtgagt ctagagcaac 20580
ccctccacac cccagatccc ctcacagcct gtagcccacc cagagccacc acccacctgc 20640
ctccggacgc agaccccacg aggttctcag caccttgccg cgtagtggcc tgcacaccgg 20700
tgttgacggc cagcccctgg tttgtctcat gctgtgtgga cttttagatt ttgcccctgc 20760
tctcttatgt acatgagcag gaatggtgag cacccagcat cagctacagg ctcctcctgg 20820
gcagcagtgg ggctcttcct gaagctgccg aggcccatgc ctgcctggga gcagtatggc 20880
caggtggact gccgagaagg ggcccacgcc tgcccgggaa tggtatggcc gggtggactg 20940
cggagaaggg gcccacgcct gcccgggaac ggtgtggccg ggtggactgc cgagaagggg 21000
cccacgcctg ccaggtggac acatcccatt ggcagcagct gtgtttcagc tccctgtggg 21060
ggcgagtagg cacagagcaa tgcccccagg gcaccagtgc ccgacctagt gggctgctga 21120
ggcgctcaga gagggaaaga gacccaggca ggaccgagct gggatagagc cacacccccg 21180
gaaggggtag gaccaggccc agctactgag acagagaact cacatgggtg cccacccaag 21240
cgggtctgtg acagagagaa tgacgcctgc aggtgtgtac aactgaccct ggggtctgaa 21300
acgcagggat tacatcccga agctgaggga ggacagagac cacatacctc caaaaagttg 21360
agaatgaaag gcacttgccc tacccccttc cctgtccctg cagtcagccc tcatcacaga 21420
ggcccctccc atcccgcaga cccctcctgc ctcgggggtg gctctgctct ctccatcttc 21480

ccggagagca ccccctgccc atcaggtgct gccccctggg cgcccctaaa tccacccaca 21540
ctcccaaccc tctcttcagg gcaggagacc ccaggaggag aggagagcgc tgaggcatgc 21600
gtggtgaggg acccagctcc acctgcctga gtctggcctg ggtgcggcag aggggcccag 21660
gccctgcacc atgggctgga ggcaggtgct cggggctgga gttgggggct gggctgcagg 21720
cctggaccct tccagggcag ggcccttgat tgagctgccc cagcccctat ggctgagctt 21780
cctggccctc tagggcaggg gccggagtgg ccatgctggg tgaacgggga aactcccgcc 21840
ctcccatggc tcactggggc cctgaatagg ccaccctgaa gccaccatcc cttcctgtgc 21900
cctcactgct gcttacagct agaaggccct ttcagaagca ccaagcccag gagagacccc 21960
tgtattgaca cctcagcctc cctggccagg gccacaccca cctcagcagc tggcttgggg 22020
cctttgcact ggcagcccct cagctcccca gtcttctccc tgctgcccag gctcccctgc 22080
tcggagccca tctctgtcct gcccaccctc tcaccacagc ccctggctga tgcagccccc 22140
tccccagcag gccgggctgg ggtgctggca cctggacacc ttcggggaga tgaagaggat 22200
gaagagcatg catcctccct ctgcctacat tgtgcggaac taaggcaggc tcctcacaat 22260
aagtgggaaa agggcatttc ttggttataa aaaggaagag gtgcccatcc aacagaggaa 22320
cctacagatc cccctaaccc agtgagtcaa gtcagcgtca tcaggaaggg gcagtccaca 22380
ccctggggca ctgcagagtc tcctccgagg ctccccaagg ctcatcatga ggaaatcctg 22440
tcccacacgg aggcccgact gcagctgatg ggctgcaccc cccagaagcg accaggtaac 22500
ggaggtgaca gaggctgagg gactccccga ttaacaggga cccacaagga gactacgagc 22560
tcaatggcag accgaatccg gatcaggagc aaacaaacaa acagctctag aaataacatg 22620
accagggcca ggtgcggtgg ctcatgcctg taatcccagc actttgggag gccgaggcgg 22680
gcggatcacc tgaggtcagg agttcgagac cagcctggcc aacaaggcga aaccccgtct 22740
ctactaaaaa tacaaacatt agcctggtgt agtggtgggt gcctataatc ccagctactc 22800
aggaggcttg aacctgggag gtggaggttg cagtgagtca agatcgcgcc accgtactcc 22860
agcctgggtg acagagcaag actccatctt aaaaaaaaaa aggagaagaa gaaagaaaaa 22920
ttattaaaaa attaaaaaga aagaacatga tgaggacaac tggaagaatc tgggcatgga 22980
ccacatcggc cccacgtgga gtctctcgag cgtggtcctg ctttgggtgt gtgcaagagt 23040
gtgccctggg ctcagccagc actctcaggt gttcaggcat caggctatct gggagagctg 23100
gagagagcac agatatgaat gtcaaggact ctggtgattt tggggcctca gatgccccct 23160
ccactgctct tgcagctttt ctacaggctt aaaattttaa accaatggtt gaggaacaga 23220
gagcacacaa aacctctgag aggctacccc tgcttccctg cggacccgtc cctgccccag 23280
ctgcgccttt gggcactgca ccatcccccc tgccccccac agcaagccag ccaggcagcc 23340
tgcatggagg ggtcactggt gggagggcac atgtttggaa ttcgacttct tcgactattt 23400
cctctaaaat ctttcctttt tcttttctct tagtgctttc caagtgccat tgccagtcat 23460
gtggcagcag agtgtcccag caagtgtctg gtgggtgaac ccctcagccc tgggtgccag 23520
ggacccaccg agcggggcca gggcctggcc tagccctccc ttggtgccat gtgaccaggc 23580
tccagcccaa gagagcgcag tgactgggct gcagggcctg cacaatgagg aaggtggggt 23640
tgtgggaggt ggaggctggc ccgctcagca gggactgttc tcagggacag gcagacctga 23700
acaaggtggt gtagacactg gaagcgctct ctgtgcctaa cccttggaag gagaccagtg 23760
tctgagcatc atctgcacct cctaaggctg gtcgattgtt tttttttctt ttttgagatg 23820
cagtctcgct cttgtccccc aggctggagt gcaatggcac gatcttggct cactgcaacc 23880
tccgcctccc gggttcaagt gattctcttg cctcagcctc ctgagtagct gggattatag 23940
gcgcctgcca ccatgcccgg ctaatttttg tatttttagt agagacgggg tttcaccatg 24000
ttggccaggc tggtctcgaa ctcctgacct caggtgatcc actcacctca acctcccaaa 24060
gtgctgggat taccggtgtg agccaccacg cccggccgaa actgccacaa actgaccgca 24120
aacaattgaa ctggtcgatc gttaatgact gctgccaaaa tgtggaccct gagtgctttg 24180
caaggtgact gcccatgctt ggcatgagtg aaggctgtct ctgagacttg atgggtcaaa 24240
caggaagatt tggggtgggt taaaaggaca gacttcagag cagtgaagga ggtgacccat 24300
gcggaagggc tattttttca agagtggaga attgcaggta aatgtttttt gtttcttggt 24360

tttgtttgtt tgagacagag tcttgctctg tcacccaggc tggagtgcag tggtacgatc 24420
tcagctcatt gtaacctctg cctcccgggt tcaagcgatt ctcctgcctc agcctcccaa 24480
gtagctggga ctacaggcat gcgccaccac acccgcttag tttttgtatc tttagtagag 24540
atggggtttt caccatgttg gccaggctgg tctcgaactc gtgacctcag gtgatccgcc 24600
ctcctcggcc tcccaaagtg cttggattac agatgtgagc caccttgcct agatggtaag 24660
tatttcaaaa cttgtggcac tgtgctgtga atgaaaaggc aggctgctca tgcctgtaat 24720
ctcagcactt tgggaggcca aggtgggtgg atcacaaggt caggagttga agaccagcct 24780
gacaatatgg tgaaaccctg tctctactaa aaatacaaaa caattagcca ggtgtggtgg 24840
cgcatgtctg taatcccagc tactcaagag gccgaggcgg gagaattgct tgaacccagg 24900
aggcagaggt tgcagtgatc agagattgtg ccaccgcact cctgcctggg ctacacagct 24960
aggctgcctc tcaaaaaaga aagaaaggca ggccgggcgc ggtggctcat gcctgtaatc 25020
acagcacttt gggaggccga ggtgggtgga tcacctgagg tcaggagttc aagaccagcc 25080
tgaccaacgt ggcaaaacac ggtctctact aaaaatacaa aaattagctg ggtgtggtgg 25140
cgggcgcctg taatcccagc tactcgggag gctgaggcag gagaatcact tgaacccggg 25200
aggcggaggt tacagtgagc caagatcatg ccactgcatt ccagcctggc gacacagcga 25260
gacttgtctc aaaaaaaaaa aaaaaaaaaa agaaaaagaa aaaagaaaag gcagaggttt 25320
cagcaggagt catgcaggga accagcttgg gactatgccc atgagtgccc ggccatgccc 25380
gcccccccag ccctggccac gcccgctgtc ctgggctccc aggctccggc cgtggtcctg 25440
caacccgcgc tttttttcca ggtggaggtc ctgattgcca taagcagcct cacgtctccg 25500
ctgctgttca cagcctctgg atatctgtca ttcagcatca tgagaatcgt ggagatgttt 25560
aaggattacc cgccagccat aaaagtgagt tgtattttat ttcagagccc cccagacatg 25620
tggtgctctg cgtgttggcc tggcccttgc tggctttgcc tgggactctg ggggcgctgg 25680
ctccagggac agctccagga ggcacctcct ccgctgttcc tgggcagcgg tgaccgtgct 25740
ccccaatgcc agcctggccc cagggcctcg gggcctcgat tccgccctgt atctggggcc 25800
accacctgct tcacagggac ctgggaccca ctctgtccct gagcccattt tagaatctgt 25860
cctgggttta tgagacatgc ttttcggatt tgcatcagaa ctgcattgag tttaacaggt 25920
gaatttaagg agaattcaaa gtcctgttac tttgagctga cccagtagga gcttatctcc 25980
aacacgactt ctgaggctca ggcacgtgta gctagatgta tttcttagat acctagtgtt 26040
tttttgtttt tttgtttttt tttttccagt ataagtggtt ttgcacccaa atcattagaa 26100
gacctgtatt aagctgtttg ctccaggtca acatgcgtgt gctgatgggg aatggagtca 26160
ctgcctgatg gggggatgga gtcactgtct gatgggggat ggagtcactg tctgatgggg 26220
gatggagtca ctgtctgatg ggggatggag tcactgtctg atggatggat ggagtcactg 26280
tctgatggat ggatggagtc actgtctgat ggaggatggg gtcactgcct gatgggggat 26340
ggagtcactg tctgatgggg gatggagtca ctgtctgatg ggggatggag tcactgatgg 26400
gggatggagt cactgcctga cgggggatgg agtcactgcc tgacgggggg atggagtcac 26460
tgcctgatgg ggggatggag tcactgcctg acagggggat ggagtcactg cctgacaggg 26520
gatagagtca ctgcctgatg gggggatgga gtcactgcct gacgggggat ggagtcactg 26580
cctgacaggg gatagagtca ctgcctgacg ggggatggag tcactgtctg acgggggatg 26640
gagtcactgt ctgacggggg atggagtcac tgtctaatgg ggtaatggag tcactgtctg 26700
atgggggatg gagtcactgt ctgatggggg catggagtca ctgtctgaag ggggatagcg 26760
tcctcggcct cccacaggtg ctgggattac aggagtgagc tacaacaccc ggcccagggt 26820
tttttcttca cttctcttgt gtttgcttca cccactccta ctccaattat tttgtttcct 26880
gtgacattta atatatctcc tacttcatgt tgtcttacaa tatgcatata attgttcacg 26940
attacaagag acaatattac cacccagggg ccatctcctg agtgagcctg aggcttccct 27000
tttatcttca gcatgcattc tgctagcagg ggacggccac acgccatcga agtcggcccc 27060
tttctgtgtg gggttatcct ctgggttcga taccccatta agtttaatgg cctctggttg 27120
tattccattt tagggcttgg tgcttttttt ctttttaaaa tggtattttt tgaatatata 27180
aaatatttat gtggttcaaa agccaaacta taaaagacga gctcagaaaa gctgtggtct 27240

catgcctgac tgacctgctc ctcttatggg caaccagatg ataatttttt tttctttctt 27300
acaaaatagc ttgtatacta gaatcactat tctgtacctt gccatttttt tttttttaag 27360
atagagtctc actctgtctc ccaggctgga gtgcagtggc acagtcttgg ctcactgcaa 27420
cctctgcctc ctgggttcaa gtgattctcc tgcctcagcc tccggagcat ctgggattac 27480
aggcatatgc caacacaccc ggctaatttt tgtactttta gcagagacag ggtttcacta 27540
tgttggccag gctggtcttg aactcctgac cttaagtgat ctgcctgtct cggcctccca 27600
aagtgctagg attacagacg tgagccaccg tgcccagcca tatacctcgc tttttagtta 27660
tgtatttatt tattttattt ttcagagaca gggtctcact ctgttgcccg ggctggtcat 27720
gagctcctgg gctgaagcga tccgcccacc ttggcctccc gaagtgctgg gattacagac 27780
gtgagccact gtgcccagcc atgtacctcg ctttttaatt atgtatttac ttattttatt 27840
tttcagagac agggtctcac tctgtcgcct gagctggtca tgagctcctg ggctcaagcg 27900
atccgcccac cttggcctcc caaagtgctg ggatgaggag gcttctgcca ccgtgcccag 27960
ccccttgctt gtttttttaa acttaataat ttatcctggt gttttctcga tacccaaggg 28020
gatcagagct cttctgaatt ccttcatcgc tcctgactgc ccggtcaacc cagccagtcc 28080
ctagtgatgg aaactgagtt atttccaacc gtctgctact ataaataact cctccgtgaa 28140
caacctgcac gcatcttcct tttgaatcct cagcgcatct ttaggatatc cagctaggag 28200
agagactgct gggtagaggg taggtgcagc tgtcatgctg ataaatctcg gcgacctcct 28260
caatgggggc cgtccacctt ccctcctgcc aatggcgtga aggtgctcac ttctccacag 28320
ccccccacag agggtgtcac gctggagttc ggtgacacgg gtgggagcac ctccgtctca 28380
atgtgggtga ggacgaggac ctcttacaaa gcatgaggac gtctgcattg ctttttcagt 28440
ggactctgct catgtctcct gaccattttc cgctaaccct ggtgccgggg gagaccagct 28500
ccatctgtga tggaggatac aaatggtggt ctcagcctgg cggctatttc ttgttctgct 28560
tgttgtgatt tttgttatgt gaaagctatt tttttaaata gttaagttga ttaatatttt 28620
ctttaatggc tctggatttt ttttttttga gactgaggaa agcataggct ttgcaataaa 28680
tggtgtctca acaaccgaag agcaaagacc acatggggcc catgccttta ccatcctcca 28740
gggtaaactc cagctgggaa cacaagtacc agactttcta ataattactt aaaatccagg 28800
ccgggcgtag tggcacatgc ctgtaatccc agcagtttgg gaggccgaag caggcagatc 28860
acctgaggtt aggagaccag cctggctaac atggtgaaac cccgtctcta ctaaaactac 28920
gaaaattagc tgggcgtggt ggcgggtacc tgtaatccca gctactggcg aggttgaggc 28980
aggagaatcg cttgaacctg ggatgtagag gttgcagcga gccgagatcg cgccactgca 29040
cgccagcctg ggtgacagag ggagactgtc gcaaaaaaaa gttttatgtt aacttgagaa 29100
gagctgttcg ttctgtttct tctcttactt tttgtagcag tgacattgga tagatgtttg 29160
gctcttttgt gtgttaacat ttagttgctt tgtttttctt gagccaacac gggaaagcta 29220
tccatgggca cagagggttt tgggtgctcc gtggtcactc tctggccccc tgcatcctcg 29280
gccgctccct ccatcctctt ccctgccgag ctcaccgcgc tgcctcctgc cccttccttc 29340
ctagcagctg gtgctcaggg gtgaagctgt tgggggtctg tgcaccccat ccccccagga 29400
cggctgcccc ctgtctcctc cgctcaggat cctgctgaca cggaacggtc agcgcttctg 29460
cctcccgcgg acattctctc tggctctggg actgagcctt gcgcgtgttg gagcaggagg 29520
ggtggagcga cttgaccact agcagccagc actgtccacg gggcctggca gtggctcggt 29580
cacttttatt cctgtatgtt gaacacaagc tgatggaatt taaagcagaa ttgacctcat 29640
taattgagct agaatgatta tcatcaggtt gttgaaatag caatgcgttt gggttactgg 29700
ggctccgact cgagccctgg cacctgctct ttgtgaaggc cgtgggcacg agggagcttt 29760
ggtctcctgg gctggagggc ggctgggcca ctctgctcac acctccttcc gcctgcagcc 29820
atcctacgat gtgctgctgc tgctgctgct gctagtgctc ctgctgcagg ccggcctcaa 29880
cacgggcacc gccatccagt gcgtgcgctt caaggtcagt gcaaggctgc agggtgcatc 29940
ctgggacacc cagaacggcc cgcaggagcg cctggctggg gaggtgagtg gcctgcaggg 30000
ggagggggca ctggggaggt gagtggccca cgggggtgcc cagccaggga ggtgagaagc 30060
ctgtggggtg gggggaagct ggggaggtga gtggcccaca ggggtgccca gccggggagg 30120

tgagtggcct gcagggggag ggggtgctgg ggaggtgagt agcccacagg ggtgcccagc 30180
cggggaggtg agtggcctgc ggtgcaggtg ctggggaggt gagtggccca tgggggcccc 30240
agccagggag gtgagaagcc tgtggggtgg gggcactggg gaggtgagtg gcccacaggg 30300
gtgctcagcc agggaggtga gaggcctgtg ggtttcaggg gtgctgggga ggtgagtggc 30360
ccacaggagc acccgctggg gaggtgagag gcctgcggtg ggggctcctg tcctggtgag 30420
ggcctccgag ggccacacga cgtggccctc gaagattcat tgtgccactt tgtattttta 30480
aaaacacccc agaaaaaccc cgaagaagta aaatgtcttt cttttctttt tttttttttt 30540
ttttgagaag gagtctcagt ctgtcaccca gactggagtg cagtggcgcg atcacggctc 30600
accacaacct ccgcctcctg ggttcaagca attctcctgc ctcagcctcc cgagtagctg 30660
ggactacagg cacccgccac caagcccagc taatttttct atttttagta gagatggggt 30720
ttcactatgt tggttagact ggtctcgaac tcctgacctc atgatccgcc tgccttggcc 30780
tcccaaagtg ctggaattac aggcgtgagc caccgcgccc agccgtaaaa tgtcttttaa 30840
aaatacaaac acaaagcctg ggtaaaccca gacccatccc tgggaaatat tctggactta 30900
atgctttcag atttgctctt agagacacag aagagggctt tgtgaggact cccacaggtc 30960
ctgaagtttc cttccctggt ctctggtggg gacgcggttc tgcagggaag gcaatgccca 31020
cccagcctgg cacatggggc tccgtaaagc tggggtgtca acaacttctg gctgtcactg 31080
cctgaacaat gttccccggg gccctgccta ctgtcttgct gcccccacat ctgaaaggca 31140
gaggctcagc gtcttccccc caccgagcta cgggtcctgg tggtgctccc ctgggcaaac 31200
ctgcccgctg accctcaggt gcagtgacca ctaggactgt caccttgatg cccaaacacc 31260
tgggcagcca ctcagcgggg ctctgagtcc tgccccacct ctaggccatc ctgggcggac 31320
cggccgctgc tcttgccctg cacaccctgc cctcctgtgt tcagcacacc accggcctca 31380
gaaccttggt gcagagcctc cagtgcccgc taagtcccgg aggacgctct cttgcccttg 31440
gctcgtttct cagctgaccc cgtcgccccc accctccacc ctccctactg ctgttccctt 31500
aacctgcctc ctcagttgct ttgctgccat ttttcctggc tgtggtcagt ctccctgcta 31560
ggacggcagt ccctgggcct gaaatgagac ccgcctgggt ttctgttgtg accagcaagg 31620
cccagagtca gacgcatgag gtgttctccc cagatcccag aactgctggt cccaggggct 31680
cagggtgggc ggctggggct cagggtgggc ggctggggct caggatggac ggctggggct 31740
caaggtgggt ggcttgggct cagggtgggc ggttggggct cagggtgggc ggctggggct 31800
cagggtggac ggctggggct cagggtggat ggctggggct tagggtggac ggctggggcc 31860
caggggctca gggtgggtgg ctggggctca gggtggacaa ctggggctca gggtggacag 31920
ctggggctca gggtggacaa ctggggctca gggtggacag ctggggctgg cgctgtcctc 31980
ccttttgcct ggaggcatct ggttacctct gccttaagtg ttttcttcct cagccgtcag 32040
gcacatgccc aaccttgggc ggcctccccc cgctccccgc cccagagcca gggcaagggg 32100
ccgagtgaag ggctgctcca tcccaggctc ggtagaaacc gtgtgtgcgg ggggggatgc 32160
aggcgtttct gggacactga ggaagtgaag gcttttatct ctttccacag aggcccaggg 32220
gttagtttcc ccacttcctt cacattcgaa gaaaacttct gaacgctggc tgctcgctgc 32280
ctgtggccga gtggcgtgtc caccttcccg ccgcacaggt ggccctggtg aagtaacacc 32340
tttcctgtct cggttgcagg tggccaggag ccccctgaag gagttcgaca aggagaaagc 32400
ctggagagcc gtcgtggtgc aaatggccca gtgaccccca gacgcggaaa ccgggtggca 32460
gcgcccagcc tggccccaag catggaaacg cacaacccct aatcgccctg agctactgct 32520
tctaacacct cttttccctt gtgtgagggc aaaccaggct gcaggtgggg ttttcacttc 32580
ctagggtagt ttaattttaa aataggccaa tgttggctag tctgtgcctc agtgagatca 32640
gtcagctccg agtggctccc gtgtcgtaac agcaggagca tggccgcaac ttcccaggcc 32700
gaggaagggc ccccggctcg gcctcttgag agccccaccc ctgaactggc cccagctcct 32760
cttcctgcct ctctcatggc ttgggctgga gtgggctctc tggacctgac cagactgtgg 32820
gtccctgcgt ctcctgccca ctctgaccgg gcttcctccc tccacgctta gggtctgtcc 32880
cgggtactca gtcagcccag tgggatctta cccacttcc[?] tgcaaggtgc acctgcccca 32940
ggctcaggct gcccagcggc tcttcctgga cagtgagagc agggctgggc gcctctgtcc 33000

tggcccggga gccgcagggg cccctcctcc agagcctggg cgcaagcgac acaggctgcc 33060
gctgctctcc caggtgaaat ccacaccagt ccacgccggg tcgcctgccc tgtctcccta 33120
cttagaccca gtcattctag agggatccac cgccacactg gccggcccac gtcctgggtg 33180
ctgtcatgcc cagcttggag tgccacgtgg ccgctgccca cgtcccgggc actgtcatgc 33240
ccagcttgga gtgccacatg gccgctgccc acgtcccggg cactgtcatg cccagcttgg 33300
agtgccacgt ggccgctgct gtgacaggca gtgttcttgg gggtggggct gcatccaagg 33360
ctttgtaaac cggctggacc acgtctccct ggccccagtg accgggggaa gctgagcccc 33420
tccctcctgt gtttgctccc attactcaaa atgcaggaca gatcaggtca gagcccagga 33480
attctcacag gttcacccag cgccctctac ctcctagcaa gtactttgtc ttgatcctca 33540
ctgagaaggc cccagggcag cggtcttctc catctccgct gttttggggt cttagggtac 33600
agcccaggcg gtcactgccc acctgccagg ctgcagggac agttgggtgt gagaataaca 33660
ctggctttgg gtagtgccat ggccaggagt gggtttccct gcgtctcctc gtcccgaggg 33720
cgcctgggtc ctcccagctg acggcagtaa atccacagtg agttggggcg actgtgaaac 33780
tggaatgctg ttactttgat aattactttc cagcaggtgt tttccttcac aatggttttg 33840
tttctttcct tctgatctga gaagacatga acgttttctc ttcaccgccg tggggtgtat 33900
tgactggtcc cccatgggct gctggaaagg cccggagatg catctgtggc ctggggccat 33960
caagatcaaa gaaccaggag gcctgggaga tgcagctgga tggggcggcc tgcagaccct 34020
gccagggggt ttgaggaccc tcccaggttt cccactgcgg aacaggagtg actctggctg 34080
ccaagatacc ttcatggtgt tcatgacaag tggaatcatt attttcaacc attgaagggg 34140
gatgcaggca agacaccttc ccagctgctc ctagagggga caagccaggc cctctctgca 34200
gtcctcggca gctccggaag gacacagtca ggggccgggc aaacactttg gccacagccc 34260
caaacaagcg ccaccgtggg agaggagagg ctgctgtcac tggtaccgga tgcagacccc 34320
accctgtctg caggccaccc ccacctccct gcagctttga ggctggcggg gtctgctcct 34380
gggaatgggg tgggagccac agggacgacc cggggcgggc tgatgtcttc ttgggggcag 34440
accagagagc tcaagtttca gagtcagaat taggcacttg gagcgttttt gctggcttgc 34500
actttcttat tttcttattt tagagcgctt aaaaaatccg gaaaaatggg gtttaaaaga 34560
actgtctctt tcagtctaca tttttgttta atacgcttga gcaataaacg ctgacttgca 34620
gacgtggndr mtrn 34634

## Claims

1. A nucleotide sequence as identified by SEQ ID. No. 1 or variants thereof, wherein one or more nucleic acids are added, substituted or deleted, with the proviso that the functional activity of the resulting polypeptide is retained.

2. The nucleotide sequence according to claim 1, extending from position no. 197 to no. 1327 of SEQ ID. No. 1.

3. A nucleotide sequence as identified by SEQ ID. No. 1, or variants thereof, wherein one or more nucleotides are added, substituted or deleted, such that the functional activity of the polypeptide encoded is essentially disturbed.

4. The nucleotide sequence according to claim 3, which is identified by SEQ ID. No. 2.

5. A genomic nucleotide sequence identified by SEQ. ID. No.5, coding for the WKL-1 polypeptide or variants thereof, wherein one or more nucleic acids are added, substituted or deleted, with the proviso that the functional activity of the polypeptide encoded thereby is essentially retained.

6. A genomic nucleotide sequence according to claim 5, wherein one or more nucleic acids are added, substituted or deleted, with the proviso that the functional activity of the polypeptide encoded thereby is essentially disturbed.

7. An oligonucleotide or cRNA, which is antisense to a nucleotide sequence according to claim 3 or 4 and which harbors a mutation as compared to the nucleic acid identified by SEQ. ID. No. 1.

8. A polypeptide encoded by a nucleic acid according to any of the claims 1 to 6.

9. The polypeptide according to claim 6, which is identified by SEQ. ID. No. 3 or SEQ. ID. No 4.

10. A pharmaceutical composition comprising a nucleic acid according to any of the claims 1 to 2 and 5 to 7.

11. A pharmaceutical composition containing a polypeptide encoded by a nucleic acid according to claim 1 or 5.

12. Use of a nucleic acid according to any of the claims 1 to 6 or parts thereof, or a polypeptide encoded by a nucleic acid according to claim 1 for the preparation of a medicament for the treatment of schizophrenic symptoms and related disorders.

13. Use of a nucleic acid according to any of the claims 1, 2 and 5 to 7 or parts thereof and/or a polypeptide according to claims 8 and 9 for screening for biologically active substances influencing symptoms of schizophrenia and related disorders.

14. The use according to claim 13, wherein the biologically active substance is a compound controlling the ion flux through the potassium channel.

15. Use of a nucleic acid according to claim 1 to 6 or parts thereof for the diagnosis of an individual's tendency to develop schizophrenia or related disorders.

16. Use of a nucleic acid according to any of the claims 1 to 6 for the preparation of a model for assessing the biologic activity of compounds interacting with a polypeptide according to claims 8 and 9.

17. The use according to claim 16, wherein the model is a cell or cell line kept in culture or a knock-in or knock-out non-human animal.

18. The use according to claim 17, wherein the non-human animal is a mouse, a rat or a monkey.

19. A recombinant cell or cell line containing a nucleic acid according to any of the claims 1 to 6.

20. A non-human animal containing a recombinant nucleic acid according to any of the claims 1 to 6.

21. Use of a cell or a cell line or a non-human animal according to any of the claims 19 or 20 for screening for biological active substances influencing schizophrenic symptoms.
